# EUROPÄISCHE PATENTSCHRIFT

(11) **EP 1 258 239 B1**
(45) Veröffentlichungstag und Bekanntmachung des Hinweises auf die Patenterteilung: **26.12.2007**
(21) Anmeldenummer: 01112127.4
(22) Anmeldetag: 17.05.2001
(51) Int. Cl.: A61K 8/02, A61K 8/22, A61K 8/39, A61K 8/73, A61K 8/81, A61K 8/25, A61Q 11/00

(54) **Chemisch aushärtendes Dental-Bleachingmaterial**
Chemically-curing dental bleaching composition
Composition de blanchissement des dents chimiquement durcissable

(43) Veröffentlichungstag der Anmeldung: 20.11.2002
(73) Patentinhaber: Kettenbach GmbH & CO. KG, 35713 Eschenburg (DE)
(72) Erfinder: Bublewitz, Alexander, Dr., 35745 Herborn (DE); Reber, Jens-Peter, Dr., 35745 Herborn (DE); Suchan, Matthias, Dr., 57627 Hachenburg (DE)
(74) Vertreter: KEIL & SCHAAFHAUSEN Patentanwälte

(56) Entgegenhaltungen:
- EP-A- 0 516 872
- WO-A-00/28955
- WO-A-82/01128
- WO-A-99/27895
- US-A- 5 240 415
- DATABASE WPI Week 199402 Derwent Publications Ltd., London, GB; AN 1994-012177 XP002179564 & JP 05 320033 A (KOMIH KK )

## Beschreibung

Die Erfindung betrifft ein Dental-Bleachingmaterial in Form eines Mehrkomponentensystems aus mindestens zwei Komponenten A und B nach Anspruch 1, das in der Lage ist, sich durch chemische Reaktion zu verfestigen, sowie Verfahren zum Bleichen von vitalen oder nicht vitalen Zähnen, Behandlung von Zahnplaque, Gingivitis und anderen oralen und periodentalen Krankheiten, die auf chemisch oxidierend wirkende Substanzen ansprechen.

In den letzten Jahren haben Verfahren zum Bleichen von Zähnen eine weite Verbreitung gefunden. Es handelt sich dabei um zahnmedizinische und kosmetische Verfahren, bei denen die Zähne durch das Einwirken geeigneter chemischer Verbindungen aufgehellt werden.

Es werden Ein- und Zweikomponentensysteme verwendet. Bei den Einkomponentensystemen ist eine Wasserstoffperoxid-Anlagerungsverbindung wie Carbamidperoxid in eine wasserfreie Matrix eingebettet. Diese besteht aus im Bereich der Galenik gängigen Substanzen wie Polyethylenglykolen (PEG). Die bekannten Bleachingmaterialien werden meistens als Gele bereitgestellt. Nach Applikation des Gels im Mundbereich bewirkt das Wasser in der Mundschleimhaut und im Speichel den Zerfall des Wasserstoffperoxid-haltigen Komplexes und führt zur langsamen Freisetzung von Wasserstoffperoxid, das als Bleichmittel wirkt.

Bei Zweikomponentensystemen (US 5928,628; US 6106,812; US 6110,446, PCT/US 99/22875) enthält eine Komponente Wasserstoffperoxid, das geliert in einer Matrix wie PEG vorliegt. Die zweite Komponente enthält hochdisperse Kieselsäure und Erdalkalicarbonate. Vor der Applikation werden die beiden Komponenten durchmischt, wobei unter anderem durch die Gegenwart von zweiwertigen Ionen wie Ca²⁺ die Zersetzung von H₂O₂ in aggressive Radikale, die eine starke Bleichwirkung haben, gefördert wird.

In PCT/US99/06146 wird eine dentale Bleichzusammensetzung beschrieben, die imstande ist, durch eine Temperaturerhöhung einen Viskositätsanstieg hervorzurufen, um ein Herauswaschen des Bleichgels während der Tragedauer im Patientenmund zu verhindern. Hierbei handelt es sich aber nicht um eine irreversible chemische Aushärtung.

Die gängigen Verfahren können vom Zahnarzt durchgeführt werden. Am häufigsten sind jedoch sogenannte "Homebleaching-Verfahren", die der Patient zu Hause durchführen kann und die unter der Kontrolle des Zahnarztes stehen. Zunächst wird dabei vom Zahnarzt mit Hilfe eines Zahntechnikers eine Schiene angefertigt. Dazu wird eine Abformung des Ober- und Unterkiefers erstellt. Die ausgehärtete Abformung wird dem Patientenmund entnommen und zur Erstellung eines Positivabdruckes mit Gips ausgegossen. Auf den fertigen Gipsabdruck wird meistens im Bereich der aufzuhellenden Zahnflächen ein Platzhaltermaterial aufgebracht. Anschließend wird im Tiefziehverfahren aus Kunststoffplatten eine Schiene hergestellt und angepasst. Das Platzhaltermaterial wird aus der fertigen Schiene entfernt, und die entstandenen Hohlräume dienen als Reservoir für ein später eingebrachtes Bleachingmaterial. Der Patient kann nun die Schiene mit nach Hause nehmen, selbstständig mit Bleachinggel befüllen und durch Einführen in den Mund für einen bestimmten Zeitraum selbstständig eine Bleichbehandlung durchführen.

Mit einem solchen Homebleaching-Verfahren muss der Patient den Zahnarzt zweimal aufsuchen, nämlich zur Abdrucknahme und zum Empfang der Schiene. Anschließend kann er die Applikation des Bleichmittels wiederholt selbstständig zu Hause durchführen.

Andererseits ist es für den Anwender noch relativ aufwendig, dass er mit der Applikation des Bleichmittels erst beginnen kann, nachdem der Zahnarzt eine Abform erstellt hat, der Zahntechniker diese über einen Gipsabdruck zu einer Schiene verarbeitet hat und der Anwender die Schiene in Empfang genommen hat. Der Anwender muss außerdem nach jeder Anwendung das klebrige Gel von der Zahnoberfläche entfernen und die Schiene säubern. Da das Gel auch in die Zahnzwischenräume eindringt, ist der Reinigungsaufwand entsprechend groß, und es ist nicht sicher gewährleistet, dass das Bleachinggel vollständig entfernt wird. Ein weiterer praktischer Nachteil ist, dass der Anwender erfahrungsgemäß dazu neigt, beim Eintragen des Gels in die Schiene zuviel Gel zu verwenden, so dass dieses beim Einbringen der Zähne in die Schiene überquillt. Die aggressive Bleichsubstanz kommt so in Kontakt mit dem Zahnfleisch, verteilt sich im Mundinnenraum und kann verschluckt werden. Durch das Einwirken auf beispielsweise freigelegte Zahnhälse ergibt sich auch die unerwünschte Nebenwirkung, dass die Sensibilität der Zähne ansteigt. Dieses Phänomen tritt häufig für einige Tage auf und ist zwar reversibel, beeinträchtigt aber trotzdem empfindlich das Wohlbefinden des Anwenders. Selbst wenn der Patient nicht zuviel Gel in die Schiene einfüllt, ermöglicht die Verwendung der beschriebenen Schiene keine Anwendung, bei der gezielt die Zähne, nicht aber das angrenzende Zahnfleisch mit dem Bleichmittel in Berührung kommt. Daher können bei dem beschriebenen Homebleaching-Verfahren aus Sicherheitsgründen nur relativ schwache Bleichmittel verwendet werden, was den Nachteil mit sich bringt, das der Anwender das Bleichmaterial öfter applizieren muss.

Ein weiterer Nachteil der bekannten Bleachingmaterialien ist, dass die Substanzen mit Bleichwirkung wie Carbamidperoxid relativ instabil sind, da sie durch Kontakt mit Wasser, z.B. mit Luftfeuchtigkeit, inaktiviert werden.

Sie müssen daher in wasserfreier Form bereitgestellt werden und sind nach Öffnen einer luftdichten Verpackung nur für eine begrenzte Zeit haltbar.

In der WO-A-99/27,895 wird ein Farbindikator für dentale Abdruckmassen beschrieben. Dieses Dokument enthält keinen Hinweis auf den Einsatz chemisch verfestigender Trägermaterialien.

Die ältere, nicht vorveröffentlichte WO-A-01/70,178 beschreibt ein Dental-Bleachingmaterial und ein Bleichverfahren für Zähne.

In der JP-A-11/116,421 wird ein Dental-Bleachingmaterial beschrieben, bei dem neben einem Bleichmittel und weiteren Materialien ein spezielles selbstaushärtendes Material eingesetzt wird.

FR-A-1,494,277 beschreibt ein Verfahren zur Fertigung von Prothesen. Dabei wird eine teilweise ausgehärtete und noch modellierbare Masse auf Acrylatbasis mit einer Flüssigkeit behandelt, welche organische Peroxide als Polymerisationsinitiator enthält.

JP-A-07/291,819 beschreibt Einkomponenten-Zusammensetzungen hergestellt durch Einmischen eines Härtungsbeschleunigers in eine Paste, die ein polymerisierbares Monomer und einen Füllstoff enthält.

US-A-4,759,798 beschreibt ausgewählte Formmassen für die Dentaltechnik.

Die der Erfindung zugrundeliegende Aufgabe ist es, Bleichmittel und Verfahren zu deren Applikation bereitzustellen, die die beschriebenen Nachteile vermeiden.

Insbesondere wäre es wünschenswert, Materialien und Verfahren bereitzustellen, die der Anwender auf einfache Art, mit hoher Spezifität für die zu bleichenden Zahnpartien und mit hoher Wirksamkeit in schonender Weise zu Hause applizieren kann. Dabei soll der Anwender möglichst wenig Zeit beim Zahnarzt aufwenden, bevor er die erste Behandlung vornehmen kann.

Die der Erfindung zugrundeliegende Aufgabe wird überraschenderweise gelöst durch ein Dental-Bleachingmaterial in Form eines Mehrkomponentensystems aus mindestens zwei Komponenten A und B, von denen die Komponente A mindestens eine Bleichsubstanz gemäß Anspruch 1 und die Komponente B mindestens ein Trägermaterial gemäß Anspruch 1 enthält, wobei das Trägermaterial durch chemische Reaktion verfestigbar ist.

Das Bleachingmaterial der Erfindung enthält als Bleichsubstanz Wasserstoffperoxid, Carbamidperoxid, Phthalimidoperoxohexansäure, Alkalipercarbonat, Alkaliperborat, Alkaliperoxodisulfat, Peroxyessigsäure, Alkalihypochlorit, Peroxide organischer Säuren oder deren Salze und/oder Anlagerungsverbindungen von Wasserstoffperoxid .

Bevorzugte Trägermaterialien sind Alginate, Alginsäuren, Acrylate, Methacrylate, Acrylsäure, Methacrylsäure, Acrylamid, Vinylacetat, N-Vinylpyrrolidone, Methylvinylethermaleat, Aziridine, Vinylether, Epoxide, Polyole, Polyamine, Di- und Polyisocyanate, Cyanacrylate, Hydroxypolydialkylsiloxane, alkenylsubstituierte Polyether und Polysiloxane, SiH- und Si-Vinyl haltige Polyether, Polysulfide, Glasionomerfüllungsmaterialien, Glasionomerzemente, Zinkphosphatzemente, Carboxylatzemente, Silicatzemente und Silicophosphatzemente.

Die Verfestigung der Trägermaterialien kann durch übliche Vernetzungs- und/oder Polymerisierungsverfahren wie ionische Vernetzung (z.B. Alginat), Verfilzung durch Kristallisation (z.B. Gips), radikalische Polymerisation (z.B. (Meth)acrylatpolymerisation), Polyaddition (z.B. Polyurethane), kationische Polymerisation (z.B. Aziridine), anionische Polymerisation (z.B. Epoxide), Hydrosilylierung und Kondensationsreaktionen (z.B. Polysulfide) erfolgen. Nach Verfestigung durch chemische Reaktion sind die Trägermaterialien vorzugsweise Calciumalginat, Polyacrylat, Polymethacrylat, Polyacrylsäure, Polyvinylpyrrolidon, Polyacrylamid, Acrylsäure-Acrylamid-Copolymerisate, Polyurethane, Polyharnstoffe, Polyethylenoxid-Polypropylenoxid-Copolymere, Polymethylvinylethermaleat, Polyamide, Polyethylenglykole und Polypropylenglykole, Polysulfide, vernetzte Polydialkylsiloxane sowie Gemische und Copolymerisate davon.

Das erfindungsgemäße Bleachingmaterial wird als Mehrkomponentensystem bereitgestellt, das zwei oder mehr Komponenten enthält. Besonders vorteilhaft ist ein Zweikomponentensystem aus zwei Komponenten A und B.

Die Komponente A enthält mindestens eine Bleichsubstanz und die Komponente B enthält mindestens eine Trägermaterial.

Besonders vorteilhaft ist es, wenn beim Vorliegen eines Zweikomponentensystems die Bleichsubstanz aus Komponente A nach dem Mischen die Verfestigung des Trägermaterials aus Komponente B katalysiert. Dies ist zum Beispiel gegeben, wenn Bleichsubstanzen wie Wasserstoffperoxid oder sonstige Peroxoverbindungen die radikalische Polymerisation von Methacrylaten oder Acrylaten katalysiert.

Die erfindungsgemäßen Bleachingmaterialien können zusätzlich verstärkende und/oder nicht verstärkende Füllstoffe, Reaktionsinhibitoren, zwei- oder dreiwertige Salze, Benetzungsmittel, Tenside, Emulgatoren, Alkohole, Wasser, Lösungsmittel, Puffersysteme, Farbstoffe, Duftstoffe, Gelbildner und/oder Substanzen zur Erhöhung der Viskosität (Verdicker und Pastenbildner) enthalten. Allgemein sind Zusatzstoffe verwendbar, von denen der Fachmann weiß, dass sie geeignet sind, die Verträglichkeit im Mundinnenraum, die Beschaffenheit (Viskosität, Austragbarkeit) oder das optische oder geschmackliche Erscheinungsbild zu verbessern oder einzustellen.

Übliche Zusatzstoffe sind auch Glykole, Glycerin, Polyethylenglykole, Propylenglykole, Diethylenglykole, Polypropylenglykole, Fettalkoholethoxylate, Polyvinylpyrrolidon, vernetztes Polyvinylpyrrolidon, Copolymerisate aus N-Vinylpyrrolidon und Vinylacetat, Polyacrylsäure oder deren Alkali- oder Erdalkalisalze, Carboxymethyl-, Methyl-, Hydroxyethyl-, Hydroxypropylcellulose und Polysaccharide, Kieselsäure, Kieselgur, Diatomeenerde, Farbstoffe, Farbpigmente und/oder Farbstoffsysteme, die durch pH-Wert- oder Redoxpotentialänderung eine Farbänderung durchlaufen.

Die erfindungsgemäßen Bleachingmaterialien und Komponenten von Zwei- oder Mehrkomponentensystemen können als Gel, Flüssigkeit, Pulver oder Paste vorliegen.

Bei Zweikomponentensystemen sind Kombinationen bevorzugt, bei denen die Komponenten A und B als Gel und Gel, Flüssigkeit und Flüssigkeit, Pulver und Pulver, Paste und Paste, Gel und Flüssigkeit, Gel und Paste, Paste und Flüssigkeit, Pulver und Paste, oder als Pulver und Flüssigkeit vorliegen.

Bei der Verwendung des erfindungsgemäßen Bleachingmaterials beim Home-bleaching-Verfahren ist dabei die einfache Anwendbarkeit von Bedeutung. Dem Anwender stehen dann vorzugsweise zwei Komponenten zur Verfügung, bei denen er in einfacher Weise mit geringem Zeit- und Kraftaufwand eine gute Durchmischung erreicht, so dass das Bleachingmaterial vor dem anschließenden Auftragen auf die Zähne möglichst homogen ist.

Die Verfestigung des Bleachingmaterials erfolgt nach dem Aufbringen auf die zu bleichenden Zähne. Nach der Verfestigung des Trägermaterials liegen die Bleachingmaterialien in einem plastischen, nicht-elastischen festen oder elastischen festen Zustand vor. Dabei ist der plastische Zustand vorzugsweise ein gelartiger oder pastöser Zustand, der elastisch feste Zustand bevorzugt ein gummiartiger Zustand und der nicht-elastisch feste Zustand bevorzugt ein zement- oder gipsartiger Zustand.

Die Erfindung betrifft auch ein Verfahren zum Bleichen von vitalen oder nicht vitalen Zähnen durch Applikation eines erfindungsgemäßen Bleachingmaterials, wobei
(a) die Komponenten des Mehrkomponentensystems gemischt werden,
(b) das Bleachingmaterial auf die Zähne, in Hohlräume der Zähne oder in das Pulpakavum appliziert wird und
(c) sich das Bleachingmaterial am Ort der Applikation verfestigt.

Bei Verwendung eines Zwei- oder Mehrkomponentensystems werden die Komponenten vermischt und anschließend auf die Zähne appliziert. Die Konsistenz des Materials sollte dabei so sein, dass es z.B. mit einem Pinsel auftragbar ist. Das Bleachingmaterial muss daher eine relativ dünne Konsistenz aufweisen und außerdem eine ausreichende Gesamtverarbeitungszeit aufweisen.

Danach verfestigt sich das Bleachingmaterial auf den Zähnen. Das erfindungsgemäße Bleachingmaterial hat den Vorteil, dass es sich aufgrund seiner Verfestigung von den Zähnen abnehmen oder, bei Vorliegen eines elastischen Zustandes, abziehen lässt. Die Zusammensetzung des Bleachingmaterials ist so zu wählen, dass das verfestigte Material zwar gut auf den Zähnen haftet, sich jedoch ohne größeren Kraftaufwand von den Zähnen abnehmen oder abziehen lässt. In der Regel lässt sich das Material in einem Stück von den Zähnen abtrennen und wird verworfen. Der Anwender muss in der Regel nicht unbedingt, wie bei der Entfernung bekannter Materialien, das Gel mit der Zahnbürste entfernen.

Vorzugsweise wird nach Verfestigung des Bleachingmaterials auf den Zähnen bzw. am Ort der Applikation eine Abformung der Zähne über dem auf den Zähnen verfestigten Bleachingmaterial genommen. Diese Abformung wird vorzugsweise wiederum mindestens einmal zur Applikation von weiterem Bleachingmaterial verwendet und erfüllt damit die Funktion einer Bleichschiene.

Die Abformung kann mit einem plastischen Kunststoffmaterial genommen werden, das die Zahnoberflächen auf der Außenseite (vestibulär) und auf der Innenseite (oral) bedeckt. Das verfestigte Bleachingmaterial wird mit abgeformt und dient als Platzhalter. Insbesondere bietet sich die Verwendung einer plastischen Kunststoffplatte oder eines Kunststoffstreifens an, der sich gut an die Oberfläche adaptieren lässt. Um eine ausreichende Verarbeitungszeit zu gewährleisten, bietet sich die Verwendung eines lichthärtenden Materials an.

Die Abformung wird von den Zähnen gelöst und dem Mund entnommen. Das verfestigte Bleachingmaterial lässt sich wiederum leicht von den Zähnen und von der Abform ablösen und wird verworfen. Die so erstellte Abform, die die Form einer Schiene hat, wird mit praxisüblichen Lichthärtegeräten zum Aushärten gebracht. Vor und nach dem Aushärten kann die Abform bzw. Schiene noch mit rotierenden Körpern bearbeitet werden, und steht dann zur Verwendung als Schiene für die Applikation von weiterem Bleachingmaterial zur Verfügung.

Die so erhaltene Schiene ist auf ideale Weise für die wiederholte Applikation von Bleachingmaterial geeignet, weil sie nicht nur den Zähnen angepasst ist, sondern auch an den Stellen, wo die Applikation von Bleachingmaterial erforderlich ist, Hohlräume aufweist, in die nach dem Befüllen und Einbringen in den Mund größere Mengen Bleachingmaterial vorliegen und wirken.

Bei dem beschriebenen Bleaching-Verfahren ist es sinnvoll, wenn der Zahnarzt die erste Auftragung des Bleachingmaterials vornimmt, und dabei in der beschriebenen Weise eine Schiene anfertigt. Diese kann direkt nach der Abformung ausgehärtet werden, und im Anschluss an die Sitzung dem Patienten mitgegeben werden, der das Bleachingmaterial dann zu Hause selbst applizieren kann.

Das erfindungsgemäße Verfahren ist mit einer bedeutenden Arbeits- und Aufwandserleichterung für den Zahnarzt und den Anwender verbunden. Das Homebleaching-Verfahren nach dem Stand der Technik bedarf einer Abformnahme, einer Modellherstellung, der Anfertigung der Schiene durch einen Zahntechniker und einer zweiten Zahnarztsitzung, bei der die Schiene übergeben und getestet wird.

Demgegenüber bekommt der Anwender bei Verwendung des erfindungsgemäßen Bleachingmaterials und Verfahrens eine Schiene bereits bei seinem ersten und einzigen Zahnarztbesuch zur Verfügung gestellt. Die Einbeziehung eines Zahntechnikers ist nicht mehr erforderlich. Darüber hinaus erfolgt mit der Erstellung der Schiene im Mund auch gleich die erste Bleich-Behandlung. Der Anwender kann somit ohne Zeitverzögerung mit dem Bleichen seiner Zähne beginnen und sich unter Umständen bereits beim ersten Zahnarztbesuch von der Wirkung des Bleachingverfahrens ein Bild machen.

Weiterhin hat das erfindungsgemäße Verfahren unter Verwendung der erfindungsgemäßen Bleachingmaterialien den Vorteil, dass durch die Beschaffenheit der erstellten Schiene, die Ausbuchtungen für Bleachingmaterial enthält, das Bleachingmaterial unter Verwendung dieser Schiene relativ spezifisch auf diejenigen Zähne oder Abschnitt der Zähne appliziert wird, die einer Bleichbehandlung bedürfen. Das Bleachingmaterial kommt nicht oder nur in geringen Mengen mit dem übrigen Mundinnenraum in Kontakt. Das erfindungsgemäße Verfahren ermöglicht eine gezielte Behandlung unter relativ niedrigem Materialeinsatz und schont das Zahnfleisch, den Mundinnenraum und die Gesundheit des Anwenders.

In einer weiteren Ausführungsform des erfindungsgemäßen Verfahrens wird das Bleachingmaterial bei der ersten Applikation unter Verwendung einer Schiene appliziert.

Das Bleachingmaterial kann außerdem nach Verfestigung einfach und sauber durch Ablösen, in der Regel in einem Stück, von den Zähnen und von der Schiene entfernt werden. Eine langwierige Befreiung der Schiene und der Zähne von klebrigen Gelresten entfällt.

Die Erfindung betrifft auch ein Verfahren zum Aufhellen verfärbter, avitaler Zähne auf Grundlage der "Walking-bleach-Technik" (T. Attin, Deutsche Zahnärztliche Zeitschrift 56 (2001), S. 78ff). Bei wurzelbehandelten Zähnen kommt es häufig zu Verfärbungen der Zahnkrone. Hierbei wird das Füllungsmaterial aus dem Pulpakavum entfernt und ein Bleichmittel appliziert. Das Bleichmittel wird einige Tage im Pulpakavum belassen und die Zugangskavität bis zum eventuellen Wechsel des Bleichmittels mit einem provisorischen Kunststoff verschlossen.

Im entsprechenden erfindungsgemäßen Verfahren wird ein, zu einem nicht elastischen, festen Zustand aushärtendes erfindungsgemäß dentales Bleachingmaterial, z.B. ein Bleachingmaterial mit einem Phosphat-, Silicat-, oder Glasionomerzement als Trägermaterial, in das Pulpakavum des zu bleichenden Zahnes eingebracht und gegenüber dem Mundinnenraum versiegelt. Dies hat gegenüber der Walking-Bleach-Technik nach dem Stand der Technik den Vorteil, dass das Bleichmittel gleichzeitig zum Bleichen und zum Verschließen des Kavums dient. Dadurch wird das Arbeitsverfahren beträchtlich erleichtert.

In einer bevorzugten Anwendungsform besteht das Bleachingmaterial aus einer Komponente A, die eine wasserhaltige Flüssigkeit oder eingedickte Paste mit einer geeigneten Konzentration an Wasserstoffperoxid ist, und einer Komponente B, die gelartig, pastös oder fest ist und ein Alginat enthält. Nach dem Mischen der Komponenten A und B werden die Calciumionen aus dem zugesetzten Calciumsulfat bzw. anderen Ca²⁺-Ionen liefernden Substanzen freigesetzt und führen zu einer ionischen Vernetzung der Alginatpolymere unter Freisetzung von Kalium- bzw. Natriumsulfat. Überraschenderweise findet diese Reaktion auch in Anwesenheit von H₂O₂ statt, so dass in akzeptablen Abbindezeiten eine ausreichende mechanische Festigkeit erreicht wird. Darüber hinaus wirken sich bei oben beschriebenen Reaktionen mehrere synergistische Effekte positiv aus:
1. Die in wässriger Lösung aus Ca²⁺ - Ionen liefernden Substanzen wie CaSO₄ freigesetzten Ca-Ionen bewirken zum einen die oben beschriebene Vernetzung der Alginate zum anderen die Aktivierung und Freisetzung von aktivem Sauerstoff aus Wasserstoffperoxid, so dass hier sehr schnell eine hohe Bleichaktivität aufgebaut wird, die zu kurzen Tragezeiten im Patientenmund führt (ca. 30 bis 240 Minuten) im Vergleich zu Einkomponentensystemen, die mehrere Stunden, bzw. über Nacht getragen werden müssen.
2. Die oben beschriebene Alginatreaktion läuft im neutralen bis alkalischen pH-Bereich ab, in dem gleichzeitig auch die Zersetzung von H₂O₂ erfolgt. Das bedeutet, dass die Bleichwirkung beim Patienten in einem neutralen bis alkalischen pH-Bereich abläuft und im Vergleich zu Bleichsystemen, die im sauren pH-Bereich wirken, eine wesentlich geringere Empfindlichkeit bzw. Sensibilität der Zähne/Zahnhälse hervorruft.
3. Die üblicherweise zur Reaktionssteuerung in Dentalalginaten eingesetzten Pyrophosphate wirken wenn sie in der H₂O₂-Komponente eingesetzt werden als Metallchelatbildner und erhöhen somit gleichzeitig die Lagerstabilität der H₂O₂-haltigen Komponente.
4. Die eingesetzten Alkalialginate dienen gleichzeitig als Verdicker bzw. Pastenbildner für ein Zweikomponenten Paste-Paste-Bleachingmaterial.
5. Wird ein Teil des oben genannten Calciumsulfats durch Calciumnitrat ersetzt, wird durch die oben beschriebene Reaktion Natrium- und Kaliumnitrat freigesetzt. Diese Salze sind dafür bekannt, die Zahnsensitivität zu reduzieren.

Weiterhin hat ein solches Bleachingmaterial gegenüber Materialien nach dem Stand der Technik auf Basis von H₂O₂-Anlagerungsverbindungen wie z.B. Carbamidperoxid den Vorteil, dass die Freisetzung des H₂O₂ nicht abhängig von der Zufuhr von Wasser aus dem Mundinnenraum ist. Daher kann das H₂O₂ gleichmäßiger und kontinuierlicher auf die Zähne einwirken als bei den bekannten Verfahren. Hingegen ist bei den bekannten Verfahren die Freisetzung von H₂O₂ davon abhängig, wie stark das applizierte Gel in der Schiene mit Feuchtigkeit in Kontakt kommt.

Im übrigen ist ein erfindungsgemäßes Bleachingmaterial, dass in einer Komponente A Wasserstoffperoxid in wässriger Lösung oder in wässrigen Gelen oder Pasten enthält, wesentlich lagerstabiler und haltbarer als bekannte Materialien, die als Bleichsubstanz H₂O₂-Anlagerungsverbindungen enthalten. Bekanntermaßen müssen solche Verbindungen wasserfrei gelagert werde, und verlieren nach einiger Zeit oder nach Kontakt mit geringen Feuchtigkeitsmengen ihre Wirkung. Dagegen sind wässrige Wasserstoffperoxidlösungen verschiedener Konzentrationen auch über lange Zeiträume stabil.

Weil das erfindungsgemäße Verfahren in besonderem Maße spezifisch und daher schonend für den Anwender ist, und weil z.B. Wasserstoffperoxid in beliebigen Konzentrationen verwendet werden kann und seine Bleichwirkung nicht von der Zufuhr von Feuchtigkeit aus der Mundschleimhaut abhängt, können die erfindungsgemäßen Bleachingmaterialien auch in relativ hohen Konzentrationen verwendet werden, ohne dass dem Anwender daraus Nachteile erwachsen. Der Anwender kann dann eine erfolgreiche Gesamtbehandlung im Rahmen von vergleichbar wenigen Einzelapplikationen erzielen. Das erfindungsgemäße Verfahren ermöglicht auch kurze Anwendungsdauern bei den einzelnen Bleachingbehandlungen. Die für viele Anwender unangenehme Applikation über Nacht ist nicht nötig.

Auch Kombinationen von Bleichsubstanzen sind möglich. So kann bei einem 2-Komponenten-Bleachingmaterial eine wässrige Komponente z.B. mit Wasserstoffperoxid versehen sein. Das H₂O₂ sorgt dafür, dass von Beginn des Bleichvorgangs eine hohe Bleichwirkung vorliegt. Die zweite wasserfreie Komponente kann zum Beispiel Carbamidperoxid enthalten, das nach Vermischen der Komponente erst langsam freigesetzt wird. Somit erhält man nach Abklingen der Initialwirkung des H₂O₂ eine Art Depotwirkung durch aus Carbamidperoxid freigesetztes Wasserstoffperoxid.

In einer möglichen Verfahrensvariante erfolgt die Aushärtung des Bleichmaterials in der Bleichschiene, die statt im Patientenmund zunächst am Gipsmodell appliziert wird. Auf diese Weise hat der Zahnarzt die Möglichkeit Menge und Verteilung des Bleachingmaterials am Modell individuell auf die Mundsituation des Patienten anzupassen. Erst nach Anpassen des ausgehärteten Bleachingmaterial, z.B. durch Beschneiden mit dem Skalpell wird dies im Patientenmund mit der Bleichschiene eingesetzt.

In einer weiteren Verfahrensvariante wird das Bleachingmaterial direkt auf die zu bleichenden Zähne aufgetragen. Nach dem Aushärten wird z.B. ein plastisches oder einpinselbares lichthärtendes Kunststoffmaterial über die mit Bleachingmaterial bedeckten Zähne modelliert bzw. aufgetragen. Nach dem Aushärten des Kunststoffmaterials erhält man auf diese Weise bereits beim ersten Bleachingvorgang eine individuell geformte Bleichschiene, die der Patient z.B. für weitere homebleaching-Maßnahmen mit nach Hause nehmen kann. Das heißt auf umständliche und indirekte Wege (wie z.B. Tiefziehverfahren) kann verzichtet werden.

Im Fall eines einfachen Pulver/Flüssigkeitssystems ist ein weiterer Vorteil der erfindungsgemäßen Bleachingmaterialien, dass sie aus preiswerten, gängigen Praxismaterialien zusammengesetzt sind. Insbesondere bei Verwendung von Wasserstoffperoxidlösungen wird eine erhebliche Kosteneinsparung gegenüber der Verwendung von wasserfreien Anlagerungsverbindungen erzielt.

Als weitere ionisch vernetzende Systeme können Zinkphosphatzemente, Carboxylatzemente, Silicatzemente, Silico-Phosphatzemente, Glasionomerzemente und Glasionomer-Füllungsmaterial eingesetzt werden. Diese Materialien werden normalerweise durch Zumischen von Wasser und Säuren zur Aushärtung gebracht. Wird das Wasser durch eine wässrige Wasserstoffperoxidlösung ersetzt, erhält man einen aushärtenden bleichaktiven Zement bzw. Füllungsmaterial.

In einer weiteren bevorzugten Ausführungsform besteht das Bleachingmaterial aus einer Komponente A, die eine wasserhaltige Flüssigkeit oder Paste mit einer geeigneten Konzentration an Bleichsubstanz ist und einer Komponente B, die flüssig, pastös oder gelartig ist und (Meth)acrylate enthält. Die Aushärtereaktion üblicher chemisch aushärtender Dentalmaterialien auf (Meth)acrylatbasis beruht auf einer radikalischen Polymerisation, die durch Vermischen des (Meth)acrylats mit einer kleinen Menge eines Radikalinitiators (z.B. Benzoylperoxid) ausgelöst wird. In diesem Fall wird ein Synergieeffekt ausgenutzt, indem ein kleiner Teil des als bleichaktive Verbindung im großen Überschuss vorhandenen Bleichsubstanz z.B. Wasserstoffperoxid nach dem Mischen der Komponenten gleichzeitig die Rolle des Initiators für die radikalische Polymerisation des (Meth)acrylats übernimmt.

Überraschenderweise verläuft die Polymerisationsreaktion trotz des großen Überschusses an Bleichsubstanz z.B. Wasserstoffperoxid in realistischen Reaktionszeiten ohne die erwarteten großen Wärmetönungen ab.

Bevorzugt werden in dieser Anwendungsform wasserlösliche (Meth)acrylate oder andere der radikalischen Polymerisation zugänglichen, ethylenisch ungesättigten Verbindungen wie Vinylether, Vinylester, N-Vinyl-, P-Vinyl-, S-Vinyl, Si-VinylVerbindungen, Allylether, Allylester oder deren Gemische eingesetzt.

In Gemischen mit den oben benannten Verbindungen sind auch normalerweise wasserunlösliche Verbindungen der oben aufgeführten Substanzklassen einsetzbar, da eine gegenseitige Solubilisierung von wasserlöslichen und wasserunlöslichen Verbindungen möglich ist.

Bevorzugt eingesetzte Verbindungen sind Acrylsäure, Methacrylsäure, Vinylsulfonsäure, Acrylamidoproparisulfonsäure, Vinylessigsäure, (Meth)allylsulfonsäure, Maleinsäure, (Meth)acrylamid, (Meth)acrylnitril, N-Vinylpyrrolidon, Vinylacetamid, Alkylaminoalkyl(meth)acrylate, Alkylaminopropylacrylamide, Acrylamidopropyltrimethylammoniumchlorid, Acrylamid, Methacrylamid, N-Vinylformamid, N-Vinylacetamid, N-Methyl-N-vinylacetamid, Acrylnitril, Methacrylnitril, Vinylformiat, Vinylacetat, Vinylpropionat, Ethylvinylether, Butylvinylether, Ester aus einwertigen C₁- bis C₁₈ Alkoholen und Acrylsäure, Methacrylsäure oder Maleinsäure, Maleinsäuremonomethylester, N-Vinylcaprolactam, Styrol, Ethylstyrol, tert-Butylstyrol, N,N'-Methylenbisacrylamid, Trimethylolpropantri(meth)acrylat, Ethylenglycoldi(meth)acrylat, Propylenglycoldi(meth)acrylat, Butandioldi(meth)acrylat, Hexandiol-di(meth)acrylat, Allylmethacrylat, zweifach- oder dreifach mit Acrylsäure oder Methacrylsäure veresterte mehrwertige Alkohole, wie Pentaerythrit oder Glycerin, Triallylamin, Tetraallylethylendiamin, Divinylbenzol, Diallylphtalat, Polyethylenglycoldivinylether mit Polyethylenglycolen einer mittleren Molmasse von 100 bis 4000 g/mol, Tri(meth)acrylate von Additionsprodukten von 6 bis 20 Mol Ethylenoxid an 1 Mol Glycerin, Pentaerythrittriallylether, Divinylharnstoff, Hydroxyethyl-(meth)acrylat, Hydroxypropyl(meth)acrylat, Hydroxybutyl(meth)acrylat, Dialkyldiallylammoniumhalogenide, Allylpiperidiniumbromid, N-Vinylimidazol, N-Vinylimidazolin, Dialkylaminoalkyl(meth)acrylate, Glycidyl(meth)acrylat, Vinylphosphonsäure, Fumarsäure, Maleinsäureanhydrid, Itaconsäure, 2-Acrylamido-2-methylpropanphosphonsäure sowie deren Amide, Hydroxyalkylester und aminogruppen- und ammoniumgruppenhaltige Ester und Amide.

Besonders bevorzugt sind Acryläure - und Methacrylsäureester von alkoxylierten einwertigen gesättigten Alkoholen, die mit 2 bis 200 Mol Ethylenoxid und/oder Propylenoxid pro Mol Alkohol umgesetzt worden sind, sowie Monoacrylsäureester und Monomethacrylsäureester von Polyethylenglycol oder Polypropylenglycol, wobei die mittleren Molmassen der Polyalkylenglycole bis zu 4000 g/mol betragen können, Polyethylenglycoldiacrylate und Polyethylenglycoldimethacrylate, sowie (Meth)acrylate und Di(meth)acrylate von Blockcopolymerisaten aus Ethylenoxid und Propylenoxid, Trimethylolpropanoxyethylattri(meth)acrylat, Pentaerythritoxyethylattetra(meth)acrylat, Glycerinethoxylattri(meth)acrylat.

Zur Beschleunigung der radikalischen Polymerisation kann der Komponente A zusätzlich zum vorhandenen Wasserstoffperoxid ein unter den Polymerisationsbedinungen in Radikale zerfallender oder Radikale erzeugender Polymerisationsinitiator zugesetzt werden, z.B. organische Peroxide und Hydroperoxide, Persulfate oder Azoverbindungen. Bevorzugt ist der Einsatz von wasserlöslichen Initiatoren oder deren Gemischen. Als Initiatoren kommen außerdem Redoxinitiatoren in Betracht. Die oxidierende Komponente des Redoxinitiators wird bevorzugt in die Komponente A des erfindungsgemäßen 2-Komponenten-Bleachingmaterials zugesetzt. Es handelt um eine der oben angegebenen Perverbindungen, insbesondere jedoch um das als Bleichmittel ohnehin in Komponente A enthaltene Wasserstoffperoxid.

Die reduzierende Komponente des Redoxininitiators wird bevorzugt der Komponente B des erfindungsgemäßen 2-Komponenten Bleachingmaterials zugesetzt. Beispielsweise werden Ascorbinsäure, Natriumascorbat, Glukose, Sorbose, Ammonium- oder Alkalihydrogensulfit, Alkalisulfit, Alkalithiosulfat, Alkalihyposulfit, Alkalipyrosulfit, Alkalisulfid, Metallionen- wie z.B. Kupfer(I)-, Eisen(II)-, Silber(I)-, Mangan(II)-haltige Metallsalze oder Natriumhydroxymethylsulfoxylat verwendet. Vorzugsweise kommen als reduzierende Komponente des Redoxinitiators, Ascorbinsäure oder Natriumsulfit zum Einsatz. Auch Gemische der genannten Redoxininitiatorsysteme sind möglich.

Der der Komponente B zugesetzte reduzierend wirkende Bestandteil des Redoxinitiators, z.B. Ascorbinsäure bewirkt gleichzeitig eine Stabilisierung des in der B-Komponente enthaltenen (Meth)acrylats (oder einer anderen ethylenisch ungesättigen Verbindung). Das Antioxidans verhindert während der Lagerzeit den schädlichen Einfluss von Luft- und Licht und verhindert eine vorzeitige Polymerisation. Erst beim Vermischen der Komponenten A und B wird z.B. die Ascorbinsäure vollständig verbraucht. Dabei entstehen aus Wasserstoffperoxid hochreaktive Hydroxylradikale, die die radikalische Polymerisation des (Meth)acrylats (oder einer anderen ethylenisch ungesättigten Verbindung) initiieren.

Durch geeignete Auswahl und Verhältnisse von Mono(meth)acrylaten und Di(meth)acrylaten und/oder mehr als zwei (Meth)acrylatgruppen enthaltenden Verbindungen lassen sich verschiedene Varianten von Bleachingmaterialien erhalten. In einer ersten Variante erhält man durch Vermischen der flüssigen Komponente A bestehend aus wässriger Wasserstoffperoxidlösung in der gewünschten Konzentration mit der flüssigen Komponente B, bestehend aus überwiegend Mono(meth)acrylaten, Wasser, Ascorbinsäure und ggf. weiteren üblichen Zusatzstoffen wie Glycerin, Farbstoffe oder Duftstoffe aus geeigneten Misch- und Dosiersystem (z.B. einer 2-Kammer-Glaskarpule) durch radikalische Polymerisation ein sich nach kurzer Verarbeitungszeit verfestigendes Bleachingmaterial, das nach vollständiger Reaktion ein gelartige Konsistenz ausbildet. Diese Variante hat den Vorteil, dass die Wasserstoffperoxid-Komponente bei beliebig einstellbarer H₂O₂-Konzentration nahezu unbegrenzt lagerstabil ist.

Dieses Material wird wie ein Bleachingmaterial nach dem Stand der Technik in einer Bleichschiene getragen. Da es sich nach Verfestigung um ein chemisch vernetztes Gel handelt, ist es jedoch im Patientenmund wesentlich weniger wasserlöslich oder auswaschbar als die physikalisch verdickten Gele nach dem Stand der Technik und damit in seiner Verträglichkeit und Wirksamkeit vorteilhaft.

In einer zweiten Variante erhält man durch Vermischen der flüssigen oder pastenförmigen Komponente A, bestehend aus Bleichsubstanz in der gewünschten Konzentration, die gegebenenfalls durch Zusatz von Polymeren verdickt werden kann, mit der flüssigen oder pastenförmigen Komponente B, bestehend aus einem Gemisch aus Mono(meth)acrylaten und Di(meth)acrylaten (und/oder mehr als zwei (Meth)acrylatgruppen enthaltende Verbindungen), Wasser, Ascorbinsäure und gegebenenfalls weitere Zusatzstoffe wie Glycerin, Kieselsäuren, Farb- oder Duftstoffe aus geeigneten Misch- und Dosiersystemen (z.B. 2-Kammerkarpulen bei flüssig-flüssig- oder Doppelkammerspritzen bei Paste-Paste-Systemen) durch radikalische Polymerisation ein Bleachingmaterial, das in einem fließenden Übergang vom flüssigen bzw. pastenartigen Zustand über einen gelartigen Zustand in einen festen vernetzten Zustand übergeht. Während der Verarbeitungszeit kann dieses Bleachingmaterial direkt auf die Zahnoberfläche aufgetragen werden. Nach dem Auftragen härtet das Material elastisch aus und kann nun zur Herstellung einer Bleichschiene, zeitsparend, direkt im Patientenmund mit einem z.B. lichthärtenden Kunststoffmaterial bedeckt werden. Gegebenenfalls kann zur Verhinderung eines Haftverbunds zwischen ausgehärtetem Bleachingmaterial und lichthärtendem Kunststoff ein Trennmittel aufgebracht werden.

In einer dritten Variante, die in ihrer Zusammensetzung und Aufteilung auf die Komponenten wie die zweite Variante aufgebaut ist, erhält man nach dem Mischen durch radikalische Polymerisation ein Bleachingmaterial, das vom flüssigen bzw. pastenartigen Zustand über einen gelartigen Zustand in einen festen, vernetzten Zustand übergeht, das vom Patienten in einer Bleichschiene getragen wird. Das Material härtet in der Bleichschiene elastisch aus und kann nach dem Bleichvorgang leicht entfernt werden.

In einer vierten Variante, die in ihrer Zusammensetzung und Aufteilung auf die Komponenten wie die zweite Variante aufgebaut ist, wobei zusätzlich in der Komponente B, Substanzen zur Lichthärtung enthalten sind, z.B. UV-Initiatoren, wie z.B. Irgacure 184 der Fa. Ciba, oder Photoinitiatoren zur Härtung mit sichtbarem Licht wie z.B. Champherchinon und ggf. Cokatalysatoren wie Aminoverbindungen, erhält man lichthärtende Bleachingmaterialien.

Das lichthärtende Bleachingsystem kann auch als Einkomponentenmaterial formuliert werden, wobei folgende Substanzen enthalten sind: Bleichsubstanz, (Meth)acrylate, UV bzw. Photoinitiator, Cokatalysatoren, Stabilisatoren sowie Hilfs- und Zusatzstoffe wie z.B. Pastenbildner und Füllstoffe.

Die beiden letztgenannten Ausführungsformen machen sich folgenden Synergieeffekt zunutze: die verwendete Lichtquelle dient einerseits zur Polymerisierung und andererseits zum Aktivieren der Bleichsubstanz.

Eine Verfahrensvariante erlaubt auch eine Aushärtung des Bleichmaterials in der Bleichschiene, die statt im Patientenmund zunächst am Gipsmodell appliziert wird. Auf diese Weise hat der Zahnarzt die Möglichkeit, Menge und Verteilung des Bleachingmaterials am Modell individuell auf die Mundsituation des Patienten anzupassen. Erst nach Anpassen des ausgehärteten Bleachingmaterials durch z.B. Beschneiden mit dem Skalpell wird dies im Patientenmund mit der Bleichschiene eingesetzt.

In einer weiteren bevorzugten Ausführungsform der Erfindung wird nach Verfestigung des Bleachingmaterials über das Bleachingmaterial eine Schicht eines Materials zur Versiegelung des Bleachingmaterials appliziert, wobei das Material kein Bleachingmaterial enthält und sich am Ort der Applikation verfestigt. Nach dem Stand der Technik sind für die Beschichtung von Zähnen und Zahnersatzteilen, in der Prophylaxe z.B. zur Fissurenversiegelung eingesetzten, am Zahn haftenden Beschichtungsmaterialien (DE 3 717 762 C2, EP 0 531 483 B1) sowie Dentinadhäsive oder Dentin/Schmelz-Adhäsive (DE 19 646 037 A1, DE 19 701 599, EP 0 266 548) bekannt. Das erfindungsgemäße aushärtende Dentalbleachingmaterial ist dabei auf ähnliche Weise wie ein am Zahn haftender Lack ausgeführt und enthält eine der beschriebenen bleichend wirkenden Zusammensetzungen. Die dem umgebenden Mundgewebe zugewandte Seite des als Lack aufgetragenen und ausgehärteten Dental-Bleachingmaterials wird mit einem weiteren, gut haftenden Lack versiegelt, der kein Bleichmittel enthält. Auf diese Weise ist gewährleistet, dass während der Tragedauer des Lacks keine Reizungen durch Herauslösen des Bleichmittels auftreten. Diese Ausführungsform ist auch unter ästhetischen Gesichtspunkten vorteilhaft, da der Patient anstelle einer sichtbaren Bleichschiene eine farblich abgestimmte oder transparenten, unauffälligen und komfortablen bleichenden Lack trägt.

Eine weitere Ausführungsform der Erfindung eines chemisch aushärtenden Dental-Bleachingmaterials basiert auf der Verwendung von Epiminen. Diese werden im Dentalbereich insbesondere als Abformmaterialien (DE-B-1544837, sowie als temporäre Kronen- und Brückenmaterialien (US-A- 3453242 und US-A-4903555) eingesetzt. Bei der in o.g. Patenten beschriebenen kationischen Polymerisation werden polyfunktionelle Imine (Synonym für Aziridin bzw. Ethylenimin-Verbindungen) zu einer elastischen bzw. harten Phase polymerisiert.

Die im erfindungsgemäßen aushärtenden dentalen Bleachingmaterial verwendeten Epimine können nach dem in DE-PS-1544837 beschriebenen Verfahren hergestellt werden. Als Vernetzer bzw. Katalysator für die kationische Polymerisation können übliche kationisierende Starter oder Aktivatoren vorzugsweise Arylsulfonsäureester, gemäß DE-PS-1544837, insbesondere 2,5-Dichlorbenzolsulfonsäuremethylestersäure, spezielle Sulfoniumsalze gemäß US-A-4167618 bzw. DE-A-2515593 verwendet werden, oder Borsäurekomplex enthaltende Katalysatorkomponenten gemäß PCT/EP00/08568.

In einer beispielhaften Ausführungsform ist die Bleichsubstanz (z.B. H₂O₂ oder Carbamidperoxid) zusammen mit dem Aktivator 2,5-Dichlorbenzolsulfonsäuremethylester als flüssige Komponente A mit Verdickern (z.B. Carbopol, PEG) bzw. mit Pastenbildnern in Pastenform formulierbar. Die Komponente B enthält vorzugsweise ein Epimin enthaltendes Polyalkylenglycol, dass in flüssiger oder Pastenform abhängig von der Molmasse des zugrundeliegenden Polyalkylenglycols oder von zugesetzten Strukturbildnern wie Kieselsäuren oder verdickenden Polymeren vorliegt. Natürlich können auch Copolymere und/oder Mischungen aus Polyethylenglycol, Polypropylenglycol und Polytetrahydrofuran eingesetzt werden. Nach dem Mischen der Komponenten A und B mit geeigneten Misch- und Dosiersystemen erhält man ein durch kationische Polymerisation aushärtendes Dental-Bleachingmaterial.

In einer weiteren erfindungsgemäßen Ausführungsform eines durch kationische Polymerisation aushärtenden Dental-Bleachingmaterials werden Vinylether verwendet. In der DE 19736471 A1, PCT/EP98/07830 und DE 19753461 A1 werden dentale Zubereitungen beschrieben, die durch kationische Polymerisation aushärten, wobei die verwendbaren kationisch polymerisierbaren Vinylether auch kommerziell erhältlich sind.

Weiterhin gibt es eine Vielzahl von Patentschriften, die den Einsatz von Vinylethern durch kationische Lichthärtung beschreiben (z.B. EP 0322808, EP 608575 A, EP 0505803 B1). Zum Einsatz kommen z.B. Tetraethylenglycoldivinylether, Polyethylenglycol-520-methylvinylether. Diese Vinylether können auch in Kombination mit Epoxiden und/oder Oxetanen eingesetzt werden.

Als Katalysator werden dabei vorzugsweise Photoinitiatoren vom Typ der Onium-Verbindungen und/oder vom Typ der Metallocenium Verbindungen jeweils mit einem komplexen Anion mit geringer Nucleophilie verwendet, insbesondere Aryliodoniumsalze wie Diphenyliodonium(tetrakis)pentafluorophenylborat, (3-toluyl)(dialkylphenyl)-iodoniumtriflat, (3-toluyl)(dialkylphenyl)iodoniumhexafluoroantimonat und/oder Sulfoniumsalze wie Triarylsulfoniumhexafluoroantimonat und Triarylsulfoniumhexafluorophosphat.

In einer beispielhaften Ausführungsform des erfindungsgemäßen aushärtenden Dental-Bleachingmaterials ist die Bleichsubstanz (z.B. Carbamidperoxid) zusammen mit dem Aktivator Diphenyliodonium(tetrakis)pentafluorophenylborat als flüssige oder pastenförmige Komponente A (ggf. mit Verdickern wie Carbopol oder Polyalkylenoxiden eingedickt) formulierbar. Die Komponente B enthält vorzugsweise ein Vinylether und/oder Oxetan und/oder Epoxid enthaltendes Polyalkylenglycol, dass in flüssiger oder Pastenform abhängig von der Molmasse des zugrundeliegenden Polyalkylenglycols oder von zugesetzten Strukturbildnern wie Kieselsäuren oder verdickenden Polymeren, vorliegt. Natürlich können auch Copolymere und/oder Mischungen aus Polyethylenglycol, Polypropylenglycol und Polytetrahydrofuran eingesetzt werden. Nach dem Mischen der Komponenten A und B mit geeigneten Misch- und Dosiersystemen erhält man ein durch kationische Polymerisation aushärtendes Dental-Bleachingmaterial.

Bei den durch kationische Polymerisation aushärtenden Dental-Bleachingmaterialien kann durch Einsatz von Persäuren, wie z.B. Phtalimidoperoxohexansäure ein Synergieeffekt genutzt werden. Einerseits wirkt die Säure als Katalysator für die kationische Polymerisation, andererseits wirkt die Persäure gleichzeitig als effektive Bleichsubstanz.

In einer weiteren Ausführungsform ist das aushärtende Dental-Bleachingmaterial als hydrophiler Polyurethanschaum ausgeführt. Aus PCT / DE 92/00192 sind Polyurethanschäume bekannt. Sie bestehen aus einem oder mehreren Polyetherpolyolen, einem oder mehreren Diisocyanaten, einem oder mehreren Superabsorbern, einem oder mehreren Beschleunigern für Polyurethane, Wasser, sowie ggf. üblichen Hilfs- und/oder Zusatzstoffen.

Das erfindungsgemäße aushärtende Dental-Bleachingmaterial auf Polyurethanbasis ist beispielsweise wie folgt aufgebaut:

Die Komponente A enthält das Bleichmittel (z.B. Wasserstoffperoxidlösung oder Carbamidperoxid) und ein Polyetherpolyol. Je nach Molmasse des Polyetherpolyols liegt die Komponente A flüssig oder pastenförmig vor. Gegebenenfalls kann die Komponente A mit einem Verdicker versetzt werden. Gegebenenfalls werden der Komponente A Beschleuniger und weitere Hilfs- und Zusatzstoffe zugefügt.

Die Komponente B enthält ein Di- oder Polyisocyanat (z.B. Hexamethylendiisocyanat, 4,4'-Diisocyanatodiphenylmethan, Toluylendiisocyanat (TDI), Hexamethylendiisocyanat-Trimer). Je nach Molmasse und Viskosität des Di- oder Polyisocyanats liegt die Komponente B flüssig oder pastenförmig vor. Gegebenenfalls kann die Komponente B mit einem Verdicker versehen werden oder mit einem Beschleuniger für Polyurethane oder weiteren Hilfs- und Zusatzstoffen versehen werden.

Nach Vermischen der Komponenten A und B aus geeigneten Misch- und Dosiersystemen (z.B. Doppelkammerspritzen) härtet das dentale Bleachingmaterial durch Polyadditionsreaktion zu einem hydrophilen bleichend wirkenden Schaum aus. Wird das o.g. System wasserfrei mit Carbamidperoxid formuliert, so entsteht kein Schaum, sondern ein gummiartiges Elastomer. Selbstverständlich kann das oben beschriebene 2-Komponenten-Polyurethan-Schaum-System auch als Einkomponenten-Polyurethan-Schaum formuliert werden, vergleichbar mit den bekannten Einkomponenten-Bauschäumen. Anstelle der monomeren Di- und Polyisocyanate können auch sogenannte Präpolymere, z.B. Isocyanatpolyether- und polyester, Biurete, Allophanate und Cyanurate eingesetzt werden, um toxikologischen Aspekten bei der Anwendung im (zahn)medizinischen Bereich gerecht zu werden.

In einer weiteren erfindungsgemäßen Ausführungsform ist das dentale Bleachingmaterial als kondensationsvernetzendes Polysulfid (Thiokol)-System ausgeführt. (Abformung in der zahnärztlichen Praxis, J.Wirz, Gustav Fischer Verlag 1993, S. 53 ff). Bei den Polysulfiden handelt es sich meist um pastenförmige Produkte, wobei die Komponente A aus Polysulfid-Polymer, z.B. Polysulfid-Polyethylenglycol und gegebenenfalls Hilfs- und Zusatzstoffen besteht, und die Viskosität durch die Kettenlänge und Struktur der Polymere einstellbar ist. Die Komponente B enthält die Bleichsubstanz (z.B. Wasserstoffperoxid, Carbamidperoxid) und gegebenenfalls als Katalysator z.B. Bleidioxid, als Vernetzer Schwefel, sowie Pastenbildner und ggf. weitere Hilfs- und Zusatzstoffe. Beim Vermischen von Komponenten A und B tritt eine Vernetzung ein, indem die funktionellen Thiolgruppen durch Oxidation kondensieren, wobei Wasser freigesetzt wird. Man erhält ein elastomeres Bleachingmaterial.

In einer weiteren erfindungsgemäßen Ausführungsform wird als abbindendes System ein kondensationsvernetzendes Silicon eingesetzt.
Die Komponente A besteht aus hydroxylendgestoppten Polydimethylsiloxanen, der Bleichsubstanz (z.B. Carbamidperoxid oder Wasserstoffperoxid), Füllstoffen und weiteren Hilfs- und Zusatzstoffen. Als Initiator für die Kondensationsreaktion werden der Komponente A geringe Mengen Wasser zugesetzt. Die Komponente B besteht aus Kieselsäureestern, einem Kondensationskatalysator (z.B. zinnorganische Verbindungen) und ggf. weiteren Hilfs- und Zusatzstoffen. Die Viskosität kann durch Pastenbildner (z.B. Kieselsäure) eingestellt werden.

Nach Mischen der Komponenten A und B erhält man ein durch Kondensationsvernetzung aushärtendes Silicon-Bleachingmaterial.

In den Druckschriften DE 4010281 A1, DE 3741575 A1 und EP 0369394 B1 werden hydrophile additionsvernetzende Polyether-Abdruckmaterialien beschrieben, die durch platinkatalysierte Hydrosilylierung aushärten. Diese Systeme können für das erfindungsgemäße aushärtende Bleachingmaterial eingesetzt werden, sie besitzen eine hydrophile Polymermatrix und bilden im ausgehärteten Zustand ein hydrophiles Vulkanisat aus. Beispielsweise besteht in einem 2-Komponentensystem die Komponente A aus einem Hydrosilylierungskatalysator (z.B. Platinkatalysator), Polyetherpolymeren mit mindestens zwei Alkenylgruppen im Molekül und der bleichaktiven Substanz (z.B. Carbamidperoxid oder Wasserstoffperoxid).

Die Viskosität der Komponente A kann über die Molmasse der Polyetherpolymeren oder durch Zusatz von Pastenbildner oder Füllstoffen eingestellt werden. Gegebenenfalls können weitere übliche Hilfs- und Zusatzstoffe zugefügt werden. Die Komponente B besteht aus einer SiH-Komponente (z.B. Polyorganowasserstoffsiloxan oder ein siloxansubstituierter Polyether mit SiH-Gruppen) und gegebenenfalls Polyetherpolymeren ohne oder mit mindestens zwei Alkenylgruppen im Molekül und gegebenenfalls weiteren Hilfs- und Zusatzstoffen. Die Viskosität der Komponente B wird über die Molmasse der zugrundeliegenden Polyetherpolymeren oder über Pastenbildner eingestellt.

Nach dem Mischen der Komponenten A und B erhält man durch hydrosilylierende Additionsvernetzung ein aushärtendes, im Endzustand elastisches, dentales Bleachingmaterial. Evtl. durch Wasserstoffentwicklung auftretende Gasbläschen beeinträchtigen das dentale Bleachingmaterial nicht in seiner Funktionsfähigkeit. Anstelle eines Elastomers entsteht in diesem Fall ein elastischer Schaum, der genauso komfortabel entnommen werden kann. Ganz allgemein gesagt sind die Anforderungen, die an ein chemisch aushärtendes Dental-Bleachingmaterial gestellt werden nicht mit den Anforderungen ähnlich aufgebauter, zugrundeliegender Dentalmaterialien, wie Abformmaterialien oder Befestigungs- und Füllungsmaterialien zu vergleichen. Wichtig ist in diesem Zusammenhang nicht, dass das abbindende Bleachingmaterial einen geringen Schrumpf oder gute mechanische Eigenschaften aufweist, vielmehr muss es zum einen eine gute Bleichwirkung aufweisen und nach 1 - 30 Minuten aushärten und im Fall der Elastomere relativ leicht in einem Stück zu entfernen sein.

### Beispiele:

### Beispiel 1a:

### Flüssige Peroxidkomponente eines durch ionische Vernetzung aushärtenden 2-Komponenten Bleachingmaterials mit Alginat als Trägermaterial.

In einem Glasgefäß werden 10 Teile 30%ige Wasserstoffperoxid-Lösung mit 40 Teilen entmineralisiertem Wasser verdünnt. Man erhält eine 6%ige Wasserstoffperoxidlösung, die in geeigneten Aufbewahrungsgebinden nahezu unbegrenzt lagerstabil ist.

### Beispiel 1b:

### Pulverförmige Alginatkomponente eines durch ionische Vernetzung aushärtenden 2-Komponenten Bleachingmaterials mit Alginat als Trägermaterial

Die Alginatkomponente wird durch Vermischen von 12 Teilen Natriumalginat, 9 Teilen Calciumsulfat, Dihydrat, 4 Teilen Magnesiumoxid, 2 Teilen Kaliumhexafluorotitanat, 0,5 Teilen Tetranatriumpyrophosphat und 72,5 Teilen Diatomeenerde hergestellt.

### Beispiel 1c:

### Durch ionische Vernetzung aushärtendes 2-Komponenten-Bleachingmaterial mit Alginat als Trägermaterial in Form eines Pulver-Flüssigkeits-Systems

11 Teile der pulverförmigen Alginatkomponente aus Beispiel 1b werden in einem Kunststoffbecher (Gipsbecher) mit 50 Teilen der 6%-igen Wasserstoffperoxidlösung aus Beispiel 1a mit Hilfe eines Kunststoffspatels innerhalb von 30 Sekunden homogen vermischt. Man erhält eine dünnfließende, standfeste Paste, die sich z.B. mit einem Pinsel gut auf Zahnoberflächen auftragen lässt. Nach 5 Minuten härtet das Bleachingmaterial weich elastisch aus. Nach Beendigung des Bleichvorgangs kann das verfestigte Material in einem Stück von den Zähnen abgenommen werden.

### Beispiel 2a:

### Pastenförmige Peroxidkomponente eines durch ionische Vernetzung aushärtenden 2-Komponenten-Bleachingmaterials mit Alginat als Trägermaterial

In einem Vakuummischer wird ein Verdicker (siehe Tabelle 1) mit 67 Teilen 30%iger Wasserstoffperoxidlösung und 13 Teilen entmineralisiertem Wasser homogen gemischt. Man erhält lagerstabile Pasten mit den in Tabelle 1 angegebenen Viskositäten und Wasserstoffperoxid-Gehalten.

**Tabelle 1:**

| Verdicker | Teile | Viskosität ¹⁾ bei 23°C | H₂O₂-Gehalt |
|---|---|---|---|
| Carbopol 934 P | 5 | 40 000 mPas | 23,6 % |
| Polyvinylpyrrolidon | 50 | 30 000 mPas | 15,0 % |
| Polyethylenglycol | 13 | 23 000 mPas | 21,6 % |
| Na-Alginat | 7 | 30 000 mPas | 23,1 % |
| Copolymerisat aus N-Vinylpyrrolidon und Vinylacetat | 50 | 40 000 mPas | 15,0 % |
| vernetztes Vinylpyrrolidon | 20 | 40 000 mPas | 20,1 % |

| | | | |
|---|---|---|---|
| ¹⁾ Die Viskosität wird mit einem Viskosimeter RS 150 der Fa. Haake bei 23°C gemessen, Oszillationsfrequenz 1Hz, Kegel-Plate-System, 35 mm, 4°-Kegel, Schubspannung 50 Pa; zur Auswertung wird der Viskositätswert nach 80 Sekunden abgelesen. | | | |

### Beispiel 2b:

### Pastenförmige Alginatkomponente eines durch ionische Vernetzung aushärtenden 2-Komponenten-Bleachingmaterials mit Alginat als Trägermaterial

In einem Vakuummischer werden 19 Teile Kaliumalginat, 18 Teile Calciumsulfat-Dihydrat, 5 Teile Magnesiumoxid, 2 Teile Tetranatriumpyrophosphat sowie Füllstoffe und Pastenbildnern gemäß Tabelle 3 zu einer homogenen Paste gemischt.

**Tabelle 2:**

| Beispiel | Pastenbildner ^{a)} | Teile Pastenbildner | Füllstoff | Teile Füllstoff |
|---|---|---|---|---|
| 2b - 1 | PEG | 40 | - | - |
| 2b - 2 | PEG | 50 | Diatomeenerde | 25 |
| 2b - 3 | PPG | 50 | Diatomeenerde | 32 |
| 2b - 4 | PPG | 36 | Quarz | 45 |
| 2b - 5 | PPG | 50 | Calciumsilicat | 32 |
| 2b - 6 | PPG | 50 | Natriumaluminiumsilikat | 32 |
| 2b - 7 | PPG | 50 | Aluminiumhydroxid | 32 |
| 2b - 8 | PPG | 50 | Aluminiumoxid | 32 |
| 2b - 9 | PPG | 50 | Titandioxid | 32 |
| 2b - 10 | PPG | 50 | Zinkoxid | 32 |
| 2b - 11 | PPG | 50 | Zeolith | 32 |
| 2b - 12 | PPG | 50 | Hydroxylapatit | 32 |
| 2b - 13 | PPG | 50 | Calciumfluorophosphat | 32 |

| | | | | |
|---|---|---|---|---|
| a) PEG entspricht Polyethylenglycol mit einer mittleren Molmasse von 400 g/mol PPG entspricht Polypropylenglycol mit einer mittleren Molmasse von 2000 g/mol. | | | | |

### Beispiel 2c:

### Durch ionische Vernetzung aushärtendes 2-Komponenten-Bleachingmaterial in Paste-Paste-Form mit Alginat als Trägermaterial

50 Teile der pastenförmigen Peroxidkomponente aus Beispiel 2a (mit Natriumalginat) und 50 Teile der pastenförmigen Alginatkomponente aus Beispiel 2b - 5 (mit Polypropylenglycol und Calciumsilikat), werden aus einer Doppelkammerspritze über einen statischen Mischer ausgetragen und homogen vermischt. Man erhält eine dünnfließende, standfeste Paste, die sich z.B. mit einem Pinsel leicht auf Zahnoberflächen auftragen lässt. Nach 5 Minuten härtet das Material weich elastisch aus und lässt sich nach dem Bleichvorgang leicht in einem Stück von den Zähnen abnehmen. Dieses Beispiel dokumentiert, dass ein Paste-Paste-System auf Alginatbasis aushärtet.

### Beispiel 3a:

### Pastenförmige Peroxidkomponente eines durch ionische Vernetzung aushärtenden 2-Komponenten-Bleachingmaterials mit Alginat als Trägermaterial.

In einem Vakuummischer wird Polyvinylpyrollidon (PVP) mit 30% iger Wasserstoffperoxidlösung und entmineralisiertem Wasser (Anteile siehe Tabelle 3) homogen gemischt.

**Tabelle 3**

| Beispiel | Teile H₂O₂ 30 %ig | Teile H₂O | Teile PVP |
|---|---|---|---|
| 3 a - 1 | 20 | 60 | 20 |
| 3 a - 2 | 30 | 50 | 20 |
| 3 a - 3 | 40 | 40 | 20 |

### Beispiel 3 b:

### Pastenförmige Alginatkomponente eines durch ionische Vernetzung aushärtenden 2-Komponenten-Bleachingmaterials mit Alginat als Trägermaterial.

In einem Vakuummischer werden 6 Teile Natriumalginat, 5 Teile hochdisperse, hydrophobierte Kieselsäure mit einer BET-Oberfläche von 140 m²/g, 5 Teile Calciumsulfat Dihydrat, 0,25 Teile Tetranatriumpyrophosphat, 50 Teile Polypropylenglycol mit einer mittleren Molmasse von 2000 g/mol ein pulverförmiges Bleichmittel und ein Füllstoff gemäß Tabelle 4 homogen gemischt.

**Tabelle 4:**

| Beispiel | Bleichmittel | Teile | Füllstoff | Teile |
|---|---|---|---|---|
| 3b - 1 | Carbamidperoxid | 28 | Calciumsilikat | 11 |
| 3b - 2 | Natriumpercarbonat | 30 | Calciumsilikat | 9 |
| 3b - 3 | Natriumperborat | 30 | Calciumsilikat | 9 |
| 3b - 4 | Kaliumperoxodisulfat | 10 | Calciumsilikat | 29 |
| 3b - 5 | Phtalimidoperoxo-hexansäure | 10 | Calciumsilikat | 29 |
| 3b - 6 | ohne | 0 | Calciumsilikat | 39 |

### Beispiel 3c:

### Durch ionische Vernetzung aushärtendes 2-Komponenten Bleachingmaterial in Paste-Paste Form mit Alginat als Trägermaterial.

80 Teile der pastenförmigen Peroxidkomponente aus Beispiel 3a-2 und 20 Teile der pastenförmigen Alginatkomponente aus Beispiel 3b-1 werden aus einer Doppelkammerspritze über einen statischen Mischer ausgetragen und homogen vermischt. Man erhält eine dünnfließende, standfeste Paste, die sich z.B. mit einem Pinsel leicht auf Zahnoberflächen auftragen lässt. Nach fünf Minuten härtet das Material weich elastisch aus und lässt sich nach dem Bleichvorgang leicht in einem Stück von den Zähnen abnehmen.

Die Druckfestigkeit des ausgehärteten Bleachingmaterials beträgt gemäß ISO 1563 0,43 MPa, die Rückstellung nach der Verformung gemäß ISO 1563 beträgt 97,0%. Diese Werte erfüllen die Mindestanforderungen der ISO 1563 für zahnärztliche Alginat-Abformmassen für die Druckfestigkeit von 0,35 MPa und für die Rückstellung nach der Verformung von 95,0%.

Das Bleichmaterial besitzt eine hohe Initial-Bleichwirkung durch das Wasserstoffperoxid und eine hohe Langzeit-Bleichwirkung durch das Carbamidperoxid. Es ist somit für das sogenannte "jump-start"-Bleaching geeignet, bei dem in der Zahnarztpraxis mit einer intensiven Behandlung die Reihe der home-bleaching-Behandlungen eröffnet wird.

In analoger Weise zum ausgeführten Beispiel 3 c können weitere Kombinationen von Peroxidpasten und Alginatpasten aus den Tabellen 3 und 4 miteinander gemischt und als aushärtendes dentales Bleachingmaterial zur Anwendung gebracht werden. Auf diese Weise lassen sich unterschiedliche Bleichintensitäten und Indikationsgebiete mit dem erfindungsgemäßen aushärtenden dentalen Bleachingmaterial erreichen.

Das Mischungsverhältnis zwischen den beiden Komponenten kann zwischen 1:1 und 1:10 variiert werden, insbesondere sind 1:1, 1:2, 1:4 und 1:5-Mischungsverhältnisse bevorzugt.

### Beispiel 4a:

### Flüssige Peroxidkomponente eines durch radikalische Polymerisation aushärtenden 2-Komponenten Bleachingmaterials mit Poly(meth)acrylaten als Trägermaterial.

Die Peroxidkomponente wird durch Verdünnen von 23 Teilen einer 30%igen Wasserstoffperoxidlösung mit 77 Teilen entmineralisiertem Wasser hergestellt. Man erhält eine 7%ige Wasserstoffperoxidlösung, die in geeigneten Aufbewahrungsgebinden nahezu unbegrenzt lagerstabil ist.

### Beispiel 4b:

### Flüssige (Meth)acrylatkomponente eines durch radikalische Polymerisation aushärtenden 2-Komponenten Bleachingmaterials mit Poly(meth)acrylaten als Trägermaterial

In einer lichtgeschützten Vakuum-Schutzgasapparatur werden 72 Teile Polyethylenglycolmonomethylethermethacrylat mit einer mittleren Molmasse von 475 g/mol, 8 Teile Polyethylenglycoldimethacrylat mit einer mittleren Molmasse von 400 g/mol, 2 Teilen Ascorbinsäure und 18 Teile Wasser unter Argon-Schutzgas zu einer homogenen Lösung gemischt. Die hergestellte Lösung wird unter Lichtausschluss gelagert.

### Beispiel 4c:

### Durch radikalische Polymerisation aushärtendes 2-Komponenten Bleachingmaterial mit Poly(meth)acrylaten als Trägermaterial in Form eines Flüssigkeits-Flüssigkeits-Systems

50 Teile der flüssigen Peroxidkomponente aus Beispiel 3a und 50 Teile der flüssigen Methacrylatkomponente aus Beispiel 3b werden z.B. in einer Glaskarpule homogen gemischt und ausgetragen. Man erhält ein Bleachingmaterial, das durch radikalische Polymerisation in einem fließenden Übergang vom flüssigen über einen gelartigen in einen festen, elastischen Zustand übergeht. Während der Verarbeitungszeit kann das Bleachingmaterial direkt auf die Zahnoberflächen aufgetragen werden, oder in eine Bleichschiene eingegeben werden. Nach Beendigung des Bleichvorgangs kann das verfestigte Material in einem Stück von den Zähnen oder aus der Bleichschiene entnommen werden.

### Beispiel 5a:

### Pastenförmige Peroxidkomponente eines durch radikalische Polymerisation aushärtenden 2-Komponenten-Bleachingmaterials mit Poly(meth)acrylaten als Trägermaterial

In einem Vakuummischer werden 67 Teile 30%ige Wasserstoffperoxidlösung mit 3 Teilen Wasser und 30 Teilen eines Polyvinylpyrrolidons mit einer Molmasse von 1.100.000 g/mol homogen gemischt. Man erhält eine farblose, transparente Paste mit einer Viskosität von 60.000 mPas.

### Beispiel 5b:

### Pastenförmige Methacrylatkomponente eines durch radikalische Polymerisation aushärtenden 2-Komponenten-Bleachingmaterials mit Poly(meth)arylaten als Trägermaterial

In einem lichtgeschützten Vakuummischer werden 55 Teile Polyethylenglycolmonomethylethermethacrylat mit einer mittleren Molmasse von 475 g/mol, 5 Teile Polyethylenglycoldimethacrylat mit einer mittleren Molmasse von 400 g/mol, und 20 Teile einer hydrophilen hochdispersen Kieselsäure mit einer BET-Oberfläche von 200 m²/g mit einer Lösung von 2 Teilen Ascorbinsäure in 18 Teilen entmineralisiertem Wasser, homogen gemischt. Man erhält eine schwach opake Paste, die unter Licht- und Luftausschluss abgefüllt und gelagert werden muss.

### Beispiel 5c:

### Durch radikalische Polymerisation aushärtendes 2-Komponenten Bleachingmaterial mit Poly(meth)acrylaten als Trägermaterial in Form eines Paste-Paste-Systems.

50 Teile der pastenförmigen Peroxidkomponente aus Beispiel 4a und 50 Teile der pastenförmigen Methacrylat-Komponente aus Beispiel 4b werden aus einer Doppelkammerspritze über einen statischen Mischer ausgetragen und homogen gemischt. Man erhält ein Bleachingmaterial, das durch radikalische Polymerisation in einem fließenden Übergang vom pastenartigen Zustand über einen gelartigen in einen festen elastischen Zustand übergeht. Während der Verarbeitungszeit kann das Bleachingmaterial direkt auf die Zahnoberflächen aufgetragen werden. Nach Beendigung des Bleichvorgangs kann das verfestigte Material in einem Stück von den Zähnen oder aus der Bleichschiene entnommen werden.

### Beispiel 6:

### Beispiel eines aushärtenden Bleachingmaterials mit einem Silico-Phosphatzement als Trägermaterial

45 Teile eines handelsüblichen Silico-Phosphatzements (Zhanelka Zinkporzellan-Zement der Fa. Speiko) werden mit 20 Teilen einer 10 %igen Wasserstoffperoxidlösung homogen gemischt. Man erhält einen opaken Zement, der als bleichende Füllung in Molaren oder Prämolaren verwendet werden kann, z.B. zum internen Bleichen von wurzelkanalbehandelten, verfärbten Zähnen.

### Beispiel 7:

### Beispiel eines aushärtenden Bleachingmaterials mit einem Polycarboxylat-Zement als Trägermaterial

50 Teile eines handelsüblichen Polycarboxylat-Zements (Poly-F-Plus der Fa. De Trey) werden mit 10 Teilen einer 10%igen Wasserstoffperoxidlösung mit einem Spatel auf einem Mischblock gemischt. Man erhält einen bleichend wirkenden Zement zur Befestigung, für Unterfüllungen, zur Grundlagenfüllung oder zur provisorischen Füllung, z.B. zum internen Bleichen von wurzelkanalbehandelten, verfärbten Zähnen.

### Beispiel 8:

### Beispiel eines aushärtenden Bleachingmaterials mit einem Glasionomerzement als Trägermaterial

30 Teile eines handelsüblichen Glasionomerzements (Aquacem der Fa. De Trey) werden mit 10 Teilen einer 10%igen Wasserstoffperoxidlösung mit einem Spatel auf einem Mischblock gemischt. Man erhält einen bleichend wirkenden Befestigungszement, z.B. zum internen Bleichen von wurzelkanalbehandelten, verfärbten Zähnen.

### Beispiel 9:

### Beispiel eines aushärtenden Bleachingmaterials mit einem Glasionomer als Trägermaterial

50 Teile eines handelsüblichen Glas-Ionomer-Füllungsmaterials (Ketac Fil Plus der Fa. Espe) werden mit 50 Teilen einer 10 %igen Wasserstoffperoxidlösung mit einem Spatel auf einem Mischblock gemischt. Man erhält ein bleichend wirkendes Zahnfüllungsmaterial, z.B. zum internen Bleichen von wurzelkanalbehandelten, verfärbten Zähnen.

### Beispiel 10:

### Beispiel zum erfindungsgemäßen Bleichverfahren

Das Bleachingmaterial gemäß Beispiel 1c wird mit Hilfe eines Pinsels in einem zusammenhängenden Strang auf die zu bleichenden Zähne aufgetragen. Fünf Minuten nach dem Auftragen härtet das Material weich elastisch aus und bleibt auf der Zahnoberfläche haften. Anschließend wird mit einem plastischen lichthärtenden Kunststoffmaterial z.B. Convertray, transparent der Fa. Wilde Dental GmbH die Zahnoberfläche des gesamten Kiefers sowohl auf der Außenseite (vestibulär), der Kaufläche und der Innenfläche (oral) bedeckt und an die Oberfläche adaptiert. Das aufgetragene ausgehärtete Bleachingmaterial dient hierbei gleichzeitig als Platzhalter. Mit einem handelsüblichen Lichthärtegerät (Typ Dent-Lite) wird die so geformte Schiene zum Aushärten gebracht.

Nach der Entnahme aus dem Mund wird die Schiene mit rotierenden Körpern bearbeitet und dem Patienten mitgegeben. Das abgebundene Bleachingmaterial lässt sich im Regelfall in einem Stück von der Zahnoberfläche entfernen oder bleibt in der Schiene hängen, aus der es sich ebenfalls leicht entfernen lässt. Der Patient erhält vom Zahnarzt eine ausreichende Menge Bleachingmaterial, das er zu Hause anmischt und in der Schiene appliziert. Üblicherweise trägt der Patient die Schiene ein- bis zweimal täglich für je 30 Minuten. Er kann nun nach jedem Wechsel das ausgehärtete Bleachingmaterial leicht entfernen.

### Beispiel 11:

### Beispiel zum erfindungsgemäßen Bleichverfahren

Zur Erstellung einer Bleichschiene wird eine Abformung des Ober- und Unterkiefers z.B. mit einem handelsüblichen Alginatabformmaterial (z.B. Xanthalgin select der Fa. Hereaus-Kulzer) vom Zahnarzt vorgenommen. Im Labor wird aufgrund dieser Abformung ein Gipsmodell hergestellt. Im Bereich der aufzuhellenden Zähne wird anschließend ein Platzhaltermaterial auf den Gips aufgebracht. Anschließend wird im Tiefziehverfahren aus Kunststoffplatten am so vorbereiteten Gipsmodell eine Schiene hergestellt und angepasst. Der Zahnarzt füllt diese Schiene mit einem der aushärtenden Bleachingmaterialien gemäß einem der Beispiele 1c, 2c, 3c oder 4c. Die mit aushärtendem Bleachingmaterial gefüllte Schiene wird zunächst am Gipsmodell appliziert. Am Gipsmodell kann eine sehr genaue Kontrolle der Menge und der Verteilung des Bleachingmaterials erfolgen. Nach dem Aushärten können überquellende Bereiche zurückgeschnitten werden. Das so bearbeitete Bleachingmaterial wird dem Patienten in der Bleichschiene im Mund appliziert. Auf diese Weise kann das Bleachingmaterial sehr gezielt und sicher an den gewünschten Stellen appliziert werden. Damit wird eine effektive, gewebeschonende und patientenfreundliche Bleichung erreicht.

### Vergleichsbeispiel 1:

30 g eines handelsüblichen Dentalsuperhartgipses Fuji Rock der Fa. GC werden in einem Vakuumgipsmischer mit 6 ml 30 %iger Wasserstoffperoxidlösung homogen gemischt. Der Gips hat eine ähnliche Konsistenz wie beim Anmischen mit 6 ml Wasser, härtet jedoch nicht aus. Nach einigen Stunden dampft vorhandenes Wasser langsam ab und es verbleibt eine feste jedoch sehr brüchige Masse, die nicht mit durch Wasser ausgehärtetem Gips vergleichbar ist.

Dieses Beispiel zeigt, dass es nicht selbstverständlich ist, dass das Wasser, bei durch Wasserzusatz härtbaren Substanzen, durch Wasserstoffperoxid oder Wasserstoffperoxidlösungen ersetzt werden kann. Vielmehr ist es überraschend, dass die Abbindereaktionen der erfindungsgemäßen Beispiele 1c, 2c, 3c und 4c durch teilweise Verwendung von Wasserstoffperoxid anstelle von Wasser nicht entscheidend beeinträchtigt werden.

## Patentansprüche (Patentansprüche für folgende(n) Vertragsstaat(en): AT, BE, CH, CY, DR, FI, GR, IE, LI, LU, MC, NL, PT, SE, TR)

1. Dental-Bleaching Material in Form eines Mehrkomponentensystems aus mindestens zwei Komponenten A und B, von denen die Komponente A mindestens eine Bleichsubstanz enthält, die ausgewählt wird aus der Gruppe bestehend aus Wasserstoffperoxid, Carbamidperoxid, Phtalimidoperoxohexansäure, Alkalipercarbonat, Alkaliperborat, Alkaliperoxodisulfat, Peroxyessigsäure, Alkalihypochlorit, Anlagerungsverbindungen von Wasserstoffperoxid und/oder Peroxide organischer Säuren und die Komponente B mindestens ein Trägermaterial enthält, das durch Verfilzung durch Kristallisation, radikalische Polymerisation, Polyaddition, kationische Polymerisation, anionische Polymerisation, Hydrosilylierung oder Kondensationsreaktionen verfestigbar ist oder das ausgewählt wird aus der Gruppe der Alginate, der Alginsäuren, der Glasionomerfüllungsmaterialien, der Glasionomerzemente, der Zinkphosphatzemente, der Carboxylatzemente, der Silicatzemente und/oder Silicophosphatzemente.

2. Bleachingmaterial nach Anspruch 1, **dadurch gekennzeichnet, dass** als Bleichsubstanz Wasserstoffperoxid und/oder Carbamidperoxid enthalten ist/sind.

3. Bleachingmaterial nach einem der Ansprüche 1 oder 2, **dadurch gekennzeichnet, dass** Kombinationen von wässrigen und wasserfreien Bleichsubstanzen eingesetzt werden, insbesondere eine Komponente enthaltend wässriges Wasserstoffperoxid und eine Komponente enthaltend wasserfreies Carbamidperoxid.

4. Bleachingmaterial nach einem der Ansprüche 1 bis 3, **dadurch gekennzeichnet, dass** als Trägermaterial durch chemische Reaktion verfestigbare Alginate, Alginsäuren, Acrylate, Methacrylate, Acrylsäure, Methacrylsäure, Acrylamid, Vinylacetat, N-Vinylpyrrolidone, Methylvinylethermaleat, Aziridine, Vinylether, Epoxide, Polyole, Polyamine, Di- und Polyisocyanate, Cyanacrylate, Hydroxypolydialkylsiloxane, alkenylsubstituierte Polyether und Polysiloxane, SiH- und Si-Vinyl haltige Polyether, Polysulfide, Glasionomerfüllungsmaterialien, Glasionomerzement, Zinkphosphatzemente, Carboxylatzemente, Silicatzemente und/oder Silicophosphatzemente enthalten sind.

5. Bleachingmaterial nach einem der Ansprüche 1 bis 4, **dadurch gekennzeichnet, dass** zusätzlich verstärkende und/oder nicht verstärkende Füllstoffe, Reaktionsinhibitoren, zwei- oder höherwertige Salze, Ca²⁺-liefernde Substanzen, Calciumsulfat, Benetzungsmittel, Tenside, Emulgatoren, Alkohole, Wasser, Lösungsmittel, Puffersysteme, Duftstoffe, Geschmacksstoffe, Gelbildner, Substanzen zur Erhöhung der Viskosität (Verdicker und Pastenbildner), Glykole, Glycerin, Polyethylenglykole, Propylenglykole, Diethylenglykole, Polypropylenglykole, Polyurethane, Fett-Alkoholethoxylate, Polyvinylpyrrolidon, Copolymerisate aus N-Vinylpyrrolidon und Vinylacetat, Polyacrylsäure oder deren Alkali- oder Erdalkalisalze, Carboxymethyl-, Methyl-, Hydroxyethyl-, Hydroxypropylcellulose, Polysaccharide, Kieselsäure, Kieselgur, Diatomeenerde, Substanzen zur Lichthärtung, UV- und Photoinitiatoren, Vernetzungskatalysatoren, Farbstoffe, Farbpigmente und/oder Farbstoffsysteme, die durch pH-Wert- oder Redoxpotentialänderung eine Farbänderung durchlaufen, enthalten sind.

6. Bleachingmaterial nach mindestens einem der Ansprüche 1 bis 5, **dadurch gekennzeichnet, dass** das Bleachingmaterial und/oder die Komponenten als Gel, Flüssigkeit, Pulver oder Paste vorliegen.

7. Bleachingmaterial nach Anspruch 6, **dadurch gekennzeichnet, dass** die Komponenten B und A in den Kombinationen Gel und Gel, Flüssigkeit und Flüssigkeit, Pulver und Pulver, Paste und Paste, Gel und Flüssigkeit, Gel und Paste, Paste und Flüssigkeit, Pulver und Paste oder Pulver und Flüssigkeit vorliegen.

8. Bleachingmaterial nach einem der Ansprüche 1 bis 7, **dadurch gekennzeichnet, dass** es nach Verfestigung des Trägermaterials in einem plastischen, einem nicht-elastischen festen oder einem elastischen festen Zustand vorliegt.

9. Verfahren zum Bleichen von vitalen oder nicht vitalen Zähnen durch Applikation eines Bleachingmaterials nach mindestens einem der Ansprüche 1 bis 8, **dadurch gekennzeichnet dass**
a) die Komponenten des Mehrkomponentensystems gemischt werden,
b) das Bleachingmaterial auf die Zähne, in Hohlräume der Zähne oder in das Pulpakavum appliziert wird und
c) sich das Bleachingmaterial am Ort der Applikation verfestigt.

10. Verfahren nach Anspruch 9, wobei nach Schritt c) in einem Schritt d) am Ort der Applikation eine Abformung der Zähne mit der Funktion einer Bleichschiene zusammen mit dem auf den Zähnen verfestigten Bleachingmaterial genommen wird.

11. Verfahren nach Anspruch 10, wobei in einem Schritt e) die genommene Abformung der Zähne mit der Funktion einer Bleichschiene mindestens einmal zur Applikation von Bleachingmaterial verwendet wird.

12. Verfahren nach Anspruch 9, **dadurch gekennzeichnet, dass** das Bleachingmaterial bei Schritt b) mit einer Schiene appliziert wird.

13. Verfahren nach Anspruch 9, **dadurch gekennzeichnet, dass** über das verfestigte Bleachingmaterial eine Schicht eines Materials zur Versiegelung des Bleachingmaterials appliziert wird, wobei das Material kein Bleachingmaterial enthält und sich am Ort der Applikation verfestigt.

14. Verwendung des Dental-Bleaching Materials nach einem der Ansprüche 1 bis 8 zum Bleichen von vitalen oder nicht vitalen Zähnen.

## Patentansprüche (Patentansprüche für folgende(n) Vertragsstaat(en): DE, ES, FR,GB, IT)

1. Dental-Bleaching Material in Form eines Mehrkomponentensystems aus mindestens zwei Komponenten A und B, von denen die Komponente A mindestens eine Bleichsubstanz enthält, die ausgewählt wird aus der Gruppe bestehend aus Wasserstoffperoxid, Carbamidperoxid, Phtalimidoperoxohexansäure, Alkalipercarbonat, Alkaliperborat, Alkaliperoxodisulfat, Peroxyessigsäure, Alkalihypochlorit, Anlagerungsverbindungen von Wasserstoffperoxid und/oder Peroxide organischer Säuren und die Komponente B mindestens ein Trägermaterial enthält, das durch Verfilzung durch Kristallisation, radikalische Polymerisation, Polyaddition, kationische Polymerisation, anionische Polymerisation, Hydrosilylierung oder Kondensationsreaktionen verfestigbar ist oder das ausgewählt wird aus der Gruppe der Alginate, der Alginsäuren, der Glasionomerfüllungsmaterialien, der Glasionomerzemente, der Zinkphosphatzemente, der Carboxylatzemente, der Silicatzemente und/oder Silicophosphatzemente, mit der Massgabe, dass Mischungen ausgeschlossen sind, die eine pulverige und eine flüssige Phase enthalten, bei denen die pulverige Phase ein Salz enthält und gegebenenfalls einen Füllstoff, wobei das Salz ausgewählt ist aus der Gruppe, die aus einem gemischten Pulver von Natrium-/Calciumsalzen des Copolymers aus Methylvinylether Maleinsäureanhydrid, einem Gemisch von gemischten Natrium- / Calciumsalzen des Copolymers aus Methylvinylether / Maleinsäureanhydrid und Mischungen davon besteht, und bei denen die flüssige Phase eine Lösung von 3 bis 50 Gew. % Wasserstoffperoxid in Wasser enthält.

2. Bleachingmaterial nach Anspruch 1, **dadurch gekennzeichnet, dass** als Bleichsubstanz Wasserstoffperoxid und/oder Carbamidperoxid enthalten ist/sind.

3. Bleachingmaterial nach einem der Ansprüche 1 oder 2, **dadurch gekennzeichnet, dass** Kombinationen von wässrigen und wasserfreien Bleichsubstanzen eingesetzt werden, insbesondere eine Komponente enthaltend wässriges Wasserstoffperoxid und eine Komponente enthaltend wasserfreies Carbamidperoxid.

4. Bleachingmaterial nach einem der Ansprüche 1 bis 3, **dadurch gekennzeichnet, dass** als Trägermaterial durch chemische Reaktion verfestigbare Alginate, Alginsäuren, Acrylate, Methacrylate, Acrylsäure, Methacrylsäure, Acrylamid, Vinylacetat, N-Vinylpyrrolidone, Methylvinylethermaleat, Aziridine, Vinylether, Epoxide, Polyole, Polyamine, Di- und Polyisocyanate, Cyanacrylate, Hydroxypolydialkylsiloxane, alkenylsubstituierte Polyether und Polysiloxane, SiH- und Si-Vinyl haltige Polyether, Polysulfide, Glasionomerfüllungsmaterialien, Glasionomerzement, Zinkphosphatzemente, Carboxylatzemente, Silicatzemente und/oder Silicophosphatzemente enthalten sind.

5. Bleachingmaterial nach einem der Ansprüche 1 bis 4, **dadurch gekennzeichnet, dass** zusätzlich verstärkende und/oder nicht verstärkende Füllstoffe, Reaktionsinhibitoren, zwei- oder höherwertige Salze, Ca²⁺-liefernde Substanzen, Calciumsulfat, Benetzungsmittel, Tenside, Emulgatoren, Alkohole, Wasser, Lösungsmittel, Puffersysteme, Duftstoffe, Geschmacksstoffe, Gelbildner, Substanzen zur Erhöhung der Viskosität (Verdicker und Pastenbildner), Glykole, Glycerin, Polyethylenglykole, Propylenglykole, Diethylenglykole, Polypropylenglykole, Polyurethane, Fett-Alkoholethoxylate, Polyvinylpyrrolidon, Copolymerisate aus N-Vinylpyrrolidon und Vinylacetat, Polyacrylsäure oder deren Alkali- oder Erdalkalisalze, Carboxymethyl-, Methyl-, Hydroxyethyl-, Hydroxypropylcellulose, Polysaccharide, Kieselsäure, Kieselgur, Diatomeenerde, Substanzen zur Lichthärtung, UV- und Photoinitiatoren, Vernetzungskatalysatoren, Farbstoffe, Farbpigmente und/oder Farbstoffsysteme, die durch pH-Wert- oder Redoxpotentialänderung eine Farbänderung durchlaufen, enthalten sind.

6. Bleachingmaterial nach einem der Ansprüche 1 bis 5, **dadurch gekennzeichnet, dass** das Bleachingmaterial und/oder die Komponenten als Gel, Flüssigkeit, Pulver oder Paste vorliegen.

7. Bleachingmaterial nach Anspruch 6, **dadurch gekennzeichnet, dass** die Komponenten B und A in den Kombinationen Gel und Gel, Flüssigkeit und Flüssigkeit, Pulver und Pulver, Paste und Paste, Gel und Flüssigkeit, Gel und Paste, Paste und Flüssigkeit, Pulver und Paste oder Pulver und Flüssigkeit vorliegen.

8. Bleachingmaterial nach einem der Ansprüche 1 bis 7, **dadurch gekennzeichnet, dass** es nach Verfestigung des Trägermaterials in einem plastischen, einem nicht-elastischen festen oder einem elastischen festen Zustand vorliegt.

9. Verfahren zum Bleichen von vitalen oder nicht vitalen Zähnen durch Applikation eines Bleachingmaterials nach mindestens einem der Ansprüche 1 bis 8, **dadurch gekennzeichnet dass**
a) die Komponenten des Mehrkomponentensystems gemischt werden,
b) das Bleachingmaterial auf die Zähne, in Hohlräume der Zähne oder in das Pulpakavum appliziert wird und
c) sich das Bleachingmaterial am Ort der Applikation verfestigt.

10. Verfahren nach Anspruch 9, wobei nach Schritt c) in einem Schritt d) am Ort der Applikation eine Abformung der Zähne mit der Funktion einer Bleichschiene zusammen mit dem auf den Zähnen verfestigten Bleachingmaterial genommen wird.

11. Verfahren nach Anspruch 10, wobei in einem Schritt e) die genommene Abformung der Zähne mit der Funktion einer Bleichschiene mindestens einmal zur Applikation von Bleachingmaterial verwendet wird.

12. Verfahren nach Anspruch 9, **dadurch gekennzeichnet, dass** das Bleachingmaterial bei Schritt b) mit einer Schiene appliziert wird.

13. Verfahren nach Anspruch 9, **dadurch gekennzeichnet, dass** über das verfestigte Bleachingmaterial eine Schicht eines Materials zur Versiegelung des Bleachingmaterials appliziert wird, wobei das Material kein Bleachingmaterial enthält und sich am Ort der Applikation verfestigt.

14. Verwendung des Dental-Bleaching Materials nach einem der Ansprüche 1 bis 8 zum Bleichen von vitalen oder nicht vitalen Zähnen.

## Claims (Claims for the following Contracting State(s): AT, BE, CH, CY, DR, FI, GR, IE, LI, LU, MC, NL, PT, SE, TR)

1. A dental bleaching material in the form of a multi-oomponent system consisting of at least two components A and B, of which component A contains at least one bleaching substance selected from the group consisting of hydrogen peroxide, carbamide peroxide, phthalimido-peroxy-caproic acid, alkali percarbonate, alkali perborate, alkali peroxodisulphate, peroxyacetic acid, alkali hypochlorite, addition compounds of hydrogen peroxide and/or peroxides of organic acids, and component B contains at least one carrier material, which can be hardened by matting as a result of crystallization, radical polymerization, polyaddition, cationic polymerization, anionic polymerization, hydrosilylation or condensation reactions, or which is selected from the group consisting of alginates, alginic acids, glass ionomer filling materials, glass ionomer cements zinc phosphate cements, carboxylate cements, silicate cements and/or silicophosphate cements.

2. A bleaching material according to Claim 1, **characterized in that** hydrogen peroxide and/or carbamide peroxide are/is contained as bleaching substance.

3. A bleaching material according to one of Claims 1 or 2, **characterized in that** combinations of aqueous and anhydrous bleaching substances are used, in particular a component containing aqueous hydrogen peroxide and a component containing anhydrous carbamide peroxide.

4. A bleaching material according to one of Claims 1 to 3, **characterized in that** also contained as a carrier material which can be hardened by chemical reaction are alginates, alginic acids, acrylates, methacrylates, acrylic acid, methacrylic acid, acrylamide, vinyl acetate, N-vinyl pyrrolidones, methyl vinyl ether maleate, aziridines, vinyl ether, epoxides, polyols, polyamines, di- and polyisocyanates, cyanoacrylates, hydroxy polydialkylsiloxanes, alkenyl-substituted polyethers and polysiloxanes, polyethers containing SiH- and Si-vinyl, polysulphides, glass ionomer filling materials, glass ionomer cement, zinc phosphate cements, carboxylate cements, silicate cements and/or silicophosphate cements.

5. A bleaching material according to one of Claims 1 to 4, **characterized in that** it also contains strengthening and/or non-strengthening fillers, reaction inhibitors, divalent salts or salts of higher valence, substances supplying Ca²⁺, calcium sulphate, wetting agents, surfactants, emulsifiers, alcohols, water, solvents, buffer systems, fragrances, flavours, gelling agents, substances for increasing viscosity (thickeners and paste-forming agents), glycols, glycerine, polyethylene glycols, propylene glycols, diethylene glycols, polypropylene glycols, polyurethanes, fatty alcohol ethoxylates, polyvinyl pyrrolidone, copolymers of N-vinyl pyrrolidone and vinyl acetate, polyacrylic acid or its alkaline or alkaline-earth salts, carboxymethyl-, methyl-, hydroxyethyl-, hydroxypropyl cellulose, polysaccharides, silicic acid, kieselguhr, diatomite, substances for light curing, UV initiators and photo-initiators, cross-linking catalysts, dyes, colour pigments and/or dye systems which undergo a colour change as a result of a change in pH value or redox potential.

6. A bleaching material according to at least one of Claims 1 to 5, **characterized in that** the bleaching material and/or the components are in the form of a gel, liquid, powder or paste.

7. A bleaching material according to Claim 6, **characterized in that** the components B and A are available in the combinations gel and gel, liquid and liquid, powder and powder, paste and paste, gel and liquid, gel and paste, paste and liquid, powder and paste or powder and liquid.

8. A bleaching material according to one of Claims 1 to 7, **characterized in that**, after hardening of the carrier material, said bleaching material is in a plastic state, a non-elastic solid state or an elastic solid state.

9. A method of bleaching vital or non-vital teeth by application of a bleaching material in accordance with at least one of Claims I to 8, **characterized in that**
a) the components of the multi-component system are mixed,
b) the bleaching material is applied to the teeth, in cavities of the teeth or in the pulp chamber, and
c) the bleaching material is hardened at the site of application.

10. A method according to Claim 9 in which, following step c), there is a step d) in which, at the site of application, an impression of the teeth having the function of a bleaching tray is made together with the bleaching material hardened on the teeth.

11. A method according to Claim 10 in which, in a step e), the impression made of the teeth having the function of a bleaching tray is used at least once for the application of bleaching material.

12. A method according to Claim 9, **characterized in that** the bleaching material is applied in step b) using a tray.

13. A method according to Claim 9, **characterized in that** a layer of material is applied over the hardened bleaching material for sealing said bleaching material, the material containing no bleaching material and being hardened at the site of application.

14. Use of the dental bleaching material according to one of Claims I to 8 for bleaching vital or non-vital teeth.

## Claims (Claims for the following Contracting State(s): DE, ES, FR, GB, IT)

1. A dental bleaching material in the form of a multi-component system consisting of at least two components A and B, of which component A contains at least one bleaching substance selected from the group consisting of hydrogen peroxide, carbamide peroxide, phthalimido-peroxy-caproic acid, alkali percarbonate, alkali perborate, alkali peroxodisulphate, peroxyacetic acid, alkali hypochlorite, addition compounds of hydrogen peroxide and/or peroxides of organic acids, and component B contains at least one carrier material, which can be hardened by matting as a result of crystallization, radical polymerization, polyaddition, cationic polymerization, anionic polymerization, hydrosilylation or condensation reactions, or which is selected from the group consisting of alginates, alginic acids, glass ionomer filling materials, glass ionomer cements zinc phosphate cements, carboxylate cements, silicate cements and/or silicophosphate cements, with the proviso that mixtures are excluded which comprise a powder and a liquid phase, in which the powder phase contains a salt and possibly a filler, the salt being selected from the group consisting of a mixed powder of sodium/calcium salts of the copolymer of methyl vinyl ether/ maleic anhydride, a mixture of mixed sodium/calcium salts of the copolymer of methyl vinyl ether/maleic anhydride and mixtures thereof, and in which the liquid phase contains a solution of 3 to 50 % by wt. of hydrogen peroxide in water.

2. A bleaching material according to Claim 1, **characterized in that** hydrogen peroxide and/or carbamide peroxide are/is contained as bleaching substance.

3. A bleaching material according to one of Claims 1 or 2, **characterized in that** combinations of aqueous and anhydrous bleaching substances are used, in particular a component containing aqueous hydrogen peroxide and a component containing anhydrous carbamide peroxide.

4. A bleaching material according to one of Claims 1 to 3, **characterized in that** also contained as a carrier material which can be hardened by chemical reaction are alginates, alginic acids, acrylates, methacrylates, acrylic acid, methacrylic acid, acrylamide, vinyl acetate, N-vinyl pyrrolidones, methyl vinyl ether maleate, aziridines, vinyl ether, epoxides, polyols, polyamines, di- and polyisocyanates, cyanoacrylates, hydroxy polydiallcylsiloxanes, alkenyl-substituted polyethers and polysiloxanes, polyethers containing SiH- and Si-vinyl, polysulphides, glass ionomer filling materials, glass ionomer cement, zinc phosphate cements, carboxylate cements, silicate cements and/or silicophosphate cements.

5. A bleaching material according to one of Claims 1 to 4, **characterized in that** it also contains strengthening and/or non-strengthening fillers, reaction inhibitors, divalent salts or salts of higher valence, substances supplying Ca²⁺, calcium sulphate, wetting agents, surfactants, emulsifiers, alcohols, water, solvents, buffer systems, fragrances, flavours, gelling agents, substances for increasing viscosity (thickeners and paste-forming agents), glycols, glycerine, polyethylene glycols, propylene glycols, diethylene glycols, polypropylene glycols, polyurethanes, fatty alcohol ethoxylates, polyvinyl pyrrolidone, copolymers of N-vinyl pyrrolidone and vinyl acetate, polyacrylic acid or its alkaline or alkaline-earth salts, carboxymethyl-, methyl-, hydroxyethyl-, hydroxypropyl cellulose, polysaccharides, silicic acid, kieselguhr, diatomite, substances for light curing, UV initiators and photo-initiators, cross-linking catalysts, dyes, colour pigments and/or dye systems which undergo a colour change as a result of a change in pH value or redox potential.

6. A bleaching material according to one of Claims 1 to 5, **characterized in that** the bleaching material and/or the components are in the form of a gel, liquid, powder or paste.

7. A bleaching material according to Claim 6, **characterized in that** the components B and A are available in the combinations gel and gel, liquid and liquid, powder and powder, paste and paste, gel and liquid, gel and paste, paste and liquid, powder and paste or powder and liquid.

8. A bleaching material according to one of Claims 1 to 7, **characterized in that**, after hardening of the carrier material, said bleaching material is in a plastic state, a non-elastic solid state or an elastic solid state.

9. A method of bleaching vital or non-vital teeth by application of a bleaching material in accordance with at least one of Claims 1 to 8, **characterized in that**
a) the components of the multi-component system are mixed,
b) the bleaching material is applied to the teeth, in cavities of the teeth or in the pulp chamber, and
c) the bleaching material is hardened at the site of application.

10. A method according to Claim 9 in which, following step c), there is a step d) in which, at the site of application, an impression of the teeth having the function of a bleaching tray is made together with the bleaching material hardened on the teeth.

11. A method according to Claim 10 in which, in a step e), the impression made of the teeth having the function of a bleaching tray is used at least once for the application of bleaching material.

12. A method according to Claim 9, **characterized in that** the bleaching material is applied in step b) using a tray.

13. A method according to Claim 9, **characterized in that** a layer of material is applied over the hardened bleaching material for sealing said bleaching material, the material containing no bleaching material and being hardened at the site of application.

14. Use of the dental bleaching material according to one of Claims 1 to 8 for bleaching vital or non-vital teeth.

## Revendications (Revendications pour l'(les) Etat(s) contractant(s) suivant(s): AT, BE, CH, CY, DR, FI, GR, IE, LI, LU, MC, NL, PT, SE, TR)

1. Matériau pour blanchir les dents sous la forme d'un système à plusieurs composants comprenant au moins deux composants A et B, parmi lesquels le composant A contient au moins une substance de blanchiment qui est choisie parmi le groupe composé de peroxyde d'hydrogène, de peroxyde de carbamide, d'acide (phtalimido)peroxyhexanoïque, de percarbonate alcalin, de perborate alcalin, de peroxydisulfate alcalin, d'acide peroxoacétique, d'hypochlorite alcalin, de composés d'addition de peroxyde d'hydrogène et/ou de peroxydes d'acides organiques, et le composant B contient au moins un matériau support qui peut être durci par feutrage, par cristallisation, polymérisation radicalaire, polyaddition, polymérisation cationique, polymérisation anionique, hydrosilylation ou par réaction de condensation ou qui est choisi dans le groupe des alginates, des acides alginiques, des matériaux de remplissage au verre ionomère, des ciments au verre ionomère, des ciments au phosphate de zinc, des ciments au carboxylate, des ciments au silicate et/ou des ciments au silicophosphate

2. Matériau pour blanchir selon la revendication 1, **caractérisé en ce que** du peroxyde d'hydrogène et/ou du peroxyde carbamide est/sont contenu(s) comme substance de blanchiment.

3. Matériau pour blanchir selon la revendication 1 ou 2, **caractérisé en ce que** des combinaisons de substances blanchissantes aqueuses et exemptes d'eau sont employées, notamment un composant contenant du peroxyde d'hydrogène aqueux et un composant contenant du peroxyde de carbamide exempt d'eau.

4. Matériau pour blanchir selon l'une des revendications 1 à 3, **caractérisé en ce que** des aglinates, des acides alginiques, des acrylates, des méthacrylates, de l'acide acrylique, de l'acide méthacrylique, de l'acrylamide, de l'acétate de vinyle, de la N-vinylpyrrolidone, du maléate de méthylvinyléther, de l'aziridine, de l'éther de vinyle, de l'époxyde, des polyols, des polyamines, des di- et polyisocyanates, des cyanoacrylates, des hydroxypolydialkylsiloxanes, du polyéther et du polysiloxane alkensubstitués, du polyéther contenant du SiH- et du Si-vinyl, des polysulfides, des matériaux de remplissage à base de verre ionomère, du ciment de verre ionomère, des ciments au phosphate de zinc, des ciments au carboxylate et/ou des ciments au silicophosphate, pouvant durcir par réaction chimique, sont contenus comme matériau support.

5. Matériau pour blanchir selon l'une des revendications 1 à 4, **caractérisé en ce que** sont également contenus des matériaux de remplissage ayant et/ou n'ayant pas d'effet de renfort, des inhibiteurs de réaction, des sels bivalents ou de valence supérieure, des substances fournissant du Ca²⁺, du sulfate de calcium, un agent mouillant, des tensioactifs, des émulsifiants, des alcools, de l'eau, un solvant, des systèmes tampon, des parfums, des aromes, des gélifiants, des substances pour augmenter la viscosité (épaississant et agents formant des pâtes), des glycols, de la glycérine, des polyéthylènes de glycol, des propylènes glycol, des diéthylènes glycol, des polypropylènes glycol, des polyuréthanes, des éthoxylates d'alcool gras, la polyvinylepyrrolidone, des copolymérisats à base de N-vinylpyrrolidone et d'acétate de vinyle, de l'acide polyacrylique ou ses sels alcalins ou alcalino-terreux, de la carboxyméthylcellulose, de la méthylcellulose, de l'hydroxypropylcellulose, des polysaccharides, de l'acide silicique, du kieselgur, de la terre de diatomée, des substances pour durcissement à la lumière, des initiateurs UV et des photoinitiateurs, des catalyseurs de réticulation, des colorants, des pigments colorés et/ou des systèmes de couleurs qui subissent un changement de couleur en cas de changement de pH ou de potentiel redox.

6. Matériau pour blanchir selon l'une des revendications 1 à 5, **caractérisé en ce que** le matériau pour blanchir et/ou les composants sont présents sous forme de gel, de liquide, de poudre ou de pâte.

7. Matériau pour blanchir selon la revendication 6, **caractérisé en ce que** les composants B et A sont présents dans les combinaisons gel et gel, liquide et liquide, poudre et poudre, pâte et pâte, gel et liquide, gel et pâte, pâte et liquide, poudre et pâte ou poudre et liquide.

8. Matériau pour blanchir selon l'une des revendications 1 à 7, **caractérisé en ce qu'**il est présent après solidification du matériau support à l'état plastique, à l'état solide non élastique ou à l'état solide élastique.

9. Procédé pour blanchir des dents vitales ou non vitales par application d'un matériau pour blanchir selon au moins l'une des revendications 1 à 8, **caractérisé en ce que**
a) les composants du système à plusieurs composants sont mélangés,
b) le matériau pour blanchir est appliqué sur les dents, dans des cavités des dents ou la cavité pulpaire et
c) le matériau pour blanchir se solidifie au lieu de l'application.

10. Procédé selon la revendication 9, dans lequel après l'étape c) une empreinte des dents est réalisée lors d'une étape d) au lieu de l'application avec pour fonction d'une gouttière de blanchiment associée au matériau pour blanchir durcit sur les dents.

11. Procédé selon la revendication 10, dans lequel l'empreinte des dents prise avec la fonction de gouttière de blanchiment est utilisée dans une étape e) au moins une fois pour l'application du matériau pour blanchir.

12. Procédé selon la revendication 9, **caractérisé en ce que** le matériau pour blanchir est appliqué à l'étape b) avec une gouttière.

13. Procédé selon la revendication 9, **caractérisé en ce qu'**une couche d'un matériau pour sceller le matériau pour blanchir est appliquée sur le matériau pour blanchir durci, le matériau ne contenant pas de matériau pour blanchir et se durcissant au lieu de l'application.

14. Utilisation du matériau pour blanchir les dents selon l'une des revendications 1 à 8 pour blanchir des dents vitales ou non vitales.

## Revendications (Revendications pour l'(les) Etat(s) contractant(s) suivant(s): DE, ES, FR, GB, IT)

1. Matériau pour blanchir les dents sous la forme d'un système à plusieurs composants comprenant au moins deux composants A et B, parmi lesquels le composant A contient au moins une substance de blanchiment qui est choisie parmi le groupe composé de peroxyde d'hydrogène, de peroxyde de carbamide, d'acide (phtalimido)peroxyhexanoïque, de percarbonate alcalin, de perborate alcalin, de peroxydisulfate alcalin, d'acide peroxoacétique, d'hypochlorite alcalin, de composés d'addition de peroxyde d'hydrogène et/ou de peroxydes d'acides organiques, et le composant B contient au moins un matériau support qui peut être durci par feutrage, par cristallisation, polymérisation radicalaire, polyaddition, polymérisation cationique, polymérisation anionique, hydrosilylation ou par réaction de condensation ou qui est choisi dans le groupe des alginates, des acides alginiques, des matériaux de remplissage au verre ionomère, des ciments au verre ionomère, des ciments au phosphate de zinc, des ciments au carboxylate, des ciments au silicate et/ou des ciments au silicophosphate, en excluant les mélanges qui contiennent une phase pulvérulente et une phase liquide dans lesquels la phase pulvérulente contient un sel et le cas échéant un matériau de remplissage, le sel étant choisi dans le groupe qui se compose d'une poudre mélangée de sels de sodium / calcium du copolymère d'éther de méthylvinyl / anhydride d'acide maléique, un mélange de sels de sodium/calcium mélangés du copolymère d'éther de méthylvinyl / anhydride d'acide maléique et de mélanges de ceux-ci, et dans lesquels la phase liquide contient une solution de 5 à 50 % en poids de peroxyde d'hydrogène dans l'eau.

2. Matériau pour blanchir selon la revendication 1, **caractérisé en ce que** du peroxyde d'hydrogène et/ou du peroxyde carbamide est/sont contenu(s) comme substance de blanchiment.

3. Matériau pour blanchir selon la revendication 1 ou 2, **caractérisé en ce que** des combinaisons de substances blanchissantes aqueuses et exemptes d'eau sont employées, notamment un composant contenant du peroxyde d'hydrogène aqueux et un composant contenant du peroxyde de carbamide exempt d'eau.

4. Matériau pour blanchir selon l'une des revendications 1 à 3, **caractérisé en ce que** des aglinates, des acides alginiques, des acrylates, des méthacrylates, de l'acide acrylique, de l'acide méthacrylique, de l'acrylamide, de l'acétate de vinyle, de la N-vinylpyrrolidone, du maléate de méthylvinyléther, de l'aziridine, de l'éther de vinyle, de l'époxyde, des polyols, des polyamines, des di- et polyisocyanates, des cyanoacrylates, des hydroxypolydialkylsiloxanes, du polyéther et du polysiloxane alkensubstitués, du polyéther contenant du SiH- et du Si-vinyl, des polysulfides, des matériaux de remplissage à base de verre ionomère, du ciment de verre ionomère, des ciments au phosphate de zinc, des ciments au carboxylate et/ou des ciments au silicophosphate, pouvant durcir par réaction chimique, sont contenus comme matériau support.

5. Matériau pour blanchir selon l'une des revendications 1 à 4, **caractérisé en ce que** sont également contenus des matériaux de remplissage ayant et/ou n'ayant pas d'effet de renfort, des inhibiteurs de réaction, des sels bivalents ou de valence supérieure, des substances fournissant du Ca²⁺, du sulfate de calcium, un agent mouillant, des tensioactifs, des émulsifiants, des alcools, de l'eau, un solvant, des systèmes tampon, des parfums, des aromes, des gélifiants, des substances pour augmenter la viscosité (épaississant et agents formant des pâtes), des glycols, de la glycérine, des polyéthylènes de glycol, des propylènes glycol, des diéthylènes glycol, des polypropylènes glycol, des polyuréthanes, des éthoxylates d'alcool gras, la polyvinylepyrrolidone, des copolymérisats à base de N-vinylpyrrolidone et d'acétate de vinyle, de l'acide polyacrylique ou ses sels alcalins ou alcalino-terreux, de la carboxyméthylcellulose, de la méthylcellulose, de l'hydroxypropylcellulose, des polysaccharides, de l'acide silicique, du kieselgur, de la terre de diatomée, des substances pour durcissement à la lumière, des initiateurs UV et des photoinitiateurs, des catalyseurs de réticulation, des colorants, des pigments colorés et/ou des systèmes de couleurs qui subissent un changement de couleur en cas de changement de pH ou de potentiel redox.

6. Matériau pour blanchir selon l'une des revendications 1 à 5, **caractérisé en ce que** le matériau pour blanchir et/ou les composants sont présents sous forme de gel, de liquide, de poudre ou de pâte.

7. Matériau pour blanchir selon la revendication 6, **caractérisé en ce que** les composants B et A sont présents dans les combinaisons gel et gel, liquide et liquide, poudre et poudre, pâte et pâte, gel et liquide, gel et pâte, pâte et liquide, poudre et pâte ou poudre et liquide.

8. Matériau pour blanchir selon l'une des revendications 1 à 7, **caractérisé en ce qu'**il est présent après solidification du matériau support à l'état plastique, à l'état solide non élastique ou à l'état solide élastique.

9. Procédé pour blanchir des dents vitales ou non vitales par application d'un matériau pour blanchir selon au moins l'une des revendications 1 à 8, **caractérisé en ce que**
a. les composants du système à plusieurs composants sont mélangés,
b. le matériau pour blanchir est appliqué sur les dents, dans des cavités des dents ou la cavité pulpaire et
c. le matériau pour blanchir se solidifie au lieu de l'application.

10. Procédé selon la revendication 9, dans lequel après l'étape c) une empreinte des dents est réalisée lors d'une étape d) au lieu de l'application avec pour fonction d'une gouttière de blanchiment associée au matériau pour blanchir durcit sur les dents.

11. Procédé selon la revendication 10, dans lequel l'empreinte des dents prise avec la fonction de gouttière de blanchiment est utilisée dans une étape e) au moins une fois pour l'application du matériau pour blanchir.

12. Procédé selon la revendication 9, **caractérisé en ce que** le matériau pour blanchir est appliqué à l'étape b) avec une gouttière.

13. Procédé selon la revendication 9, **caractérisé en ce qu'**une couche d'un matériau pour sceller le matériau pour blanchir est appliquée sur le matériau pour blanchir durci, le matériau ne contenant pas de matériau pour blanchir et se durcissant au lieu de l'application.

14. Utilisation du matériau pour blanchir les dents selon l'une des revendications 1 à 8 pour blanchir des dents vitales ou non vitales.
